(19) **Europäisches Patentamt**
**European Patent Office**
**Office européen des brevets**

(11) **EP 4 349 388 B1**

(12) **EUROPÄISCHE PATENTSCHRIFT**

(45) Veröffentlichungstag und Bekanntmachung des Hinweises auf die Patenterteilung:
**27.08.2025 Patentblatt 2025/35**

(21) Anmeldenummer: **23199944.2**

(22) Anmeldetag: **27.09.2023**

(51) Internationale Patentklassifikation (IPC):
**A61M 16/00** $^{(2006.01)}$

(52) Gemeinsame Patentklassifikation (CPC):
**A61M 16/024; A61M 16/1005;** A61M 16/0066;
A61M 16/0078; A61M 16/06; A61M 16/0808;
A61M 16/0833; A61M 16/085; A61M 16/0891;
A61M 16/104; A61M 16/1075; A61M 16/18;
A61M 16/205; A61M 16/208; A61M 16/209; (Forts.)

(54) **VORRICHTUNG UND VERFAHREN ZUR DETEKTION EINES LECKS WÄHREND EINER KÜNSTLICHEN BEATMUNG**

APPARATUS AND METHOD FOR DETECTING A LEAK DURING ARTIFICIAL RESPIRATION

APPAREIL ET PROCÉDÉ DE DÉTECTION DE FUITE PENDANT UNE VENTILATION ARTIFICIELLE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **05.10.2022 DE 102022125601**

(43) Veröffentlichungstag der Anmeldung:
**10.04.2024 Patentblatt 2024/15**

(73) Patentinhaber: **Drägerwerk AG & Co. KGaA**
**23558 Lübeck (DE)**

(72) Erfinder:
• **Jahns, Robert**
  **23558 Lübeck (DE)**
• **Dicks, Bernd-Michael**
  **23558 Lübeck (DE)**
• **Peter, Gerd**
  **23558 Lübeck (DE)**
• **Heise, Tobias**
  **23558 Lübeck (DE)**
• **Kroh, Martin**
  **23558 Lübeck (DE)**

(56) Entgegenhaltungen:
DE-A1- 102010 014 883    DE-A1- 102020 002 570
DE-A1- 102020 117 607    DE-U1- 29 613 243
US-A- 2 916 033          US-A1- 2015 374 947

(52) Gemeinsame Patentklassifikation (CPC): (Forts.)
A61M 2016/0027; A61M 2016/1025;
A61M 2016/103; A61M 2016/1035; A61M 2205/15;
A61M 2205/3317; A61M 2205/3368;
A61M 2230/432; A61M 2230/435; A61M 2230/437;
G01R 33/00

**Beschreibung**

Die Erfindung betrifft eine Vorrichtung und ein Verfahren, welche während einer künstlichen Beatmung eines Patienten ein Leck, insbesondere ein schlagartig auftretendes Leck, automatisch zu detektieren vermögen. Während der künstlichen Beatmung wird eine Gasprobe aus einer Patienten-Fluidführungseinheit abgezweigt und zu einer Sensor-Anordnung geleitet, - Anordnung geleitet, welche die Gasprobe analysiert. Das Leck kann Messungen der Sensor-Anordnung verfälschen.

Der Erfindung liegt die Aufgabe zugrunde, ein Überwachungs-Verfahren zur Überwachung eines Messsystems bereitzustellen, wobei das Messsystem eine Gasprobe aus einer Beatmungs-Anordnung zur künstlichen Beatmung eines Patienten zu untersuchen vermag und das Überwachungs-Verfahren relativ zuverlässig ein schlagartig auftretendes Leck automatisch zu detektieren vermag. Weiterhin liegt der Erfindung die Aufgabe zugrunde, ein Messsystem bereitzustellen, welches eine Gasprobe zu untersuchen und relativ zuverlässig ein schlagartig auftretendes Leck automatisch zu detektieren vermag.

Die Aufgabe wird durch ein Überwachungs-Verfahren mit den Merkmalen des Anspruchs 1 und durch ein Messsystem mit den Merkmalen des Anspruchs 9 gelöst. Vorteilhafte Ausgestaltungen des erfindungsgemäßen Überwachungs-Verfahrens sind, soweit sinnvoll, auch vorteilhafte Ausgestaltungen des erfindungsgemäßen Messsystems und umgekehrt.

Das erfindungsgemäße Messsystem ist dazu ausgestaltet, bei der künstlichen Beatmung eines Patienten verwendet zu werden. Der Patient ist mit einer patientenseitigen Koppeleinheit verbunden oder lässt sich wenigstens zeitweise mit einer patientenseitigen Koppeleinheit verbinden. Eine Atemmaske, ein Tubus und ein Katheter sind Beispiele für eine patientenseitige Koppeleinheit.

Wenigstens zeitweise während dieser künstlichen Beatmung ist eine Fluidverbindung zwischen der patientenseitigen Koppeleinheit und einem medizinischen Gerät hergestellt. Das medizinische Gerät ist oder umfasst bevorzugt ein Beatmungsgerät, welches ein Gasgemisch für die künstliche Beatmung erzeugt und durch die Fluidverbindung zur patientenseitigen Koppeleinheit fördert. Das medizinische Gerät kann auch einen Handbeatmungsbeutel umfassen. Das Gasgemisch enthält Sauerstoff und optional mindestens ein Narkosemittel. Die Fluidverbindung ist mithilfe einer Patienten-Fluidführungseinheit hergestellt.

Mit dem erfindungsgemäßen Überwachungs-Verfahren lässt sich ein solches Messsystem überwachen. Das Überwachungs-Verfahren wird durchgeführt, während die Fluidverbindung zwischen der patientenseitige Koppeleinheit und dem medizinischen Gerät hergestellt ist. Das erfindungsgemäße Messsystem vermag sich selbst aus ein Leck zu überwachen.

Das erfindungsgemäße Messsystem umfasst eine Sensor-Anordnung und eine Sensor-Fluidführungseinheit, die wenigstens zeitweise in einer Fluidverbindung mit der Patienten-Fluidführungseinheit und mit der Sensor-Anordnung steht. Die Sensor-Anordnung umfasst einen Wärmeleitfähigkeits-Sensor und eine Signalverarbeitungseinheit.

Das erfindungsgemäße Überwachungs-Verfahren umfasst die folgenden automatisch durchgeführten Schritte, und das erfindungsgemäße Messsystem ist dazu ausgestaltet, automatisch die folgenden Schritte durchzuführen:

- Wenigstens zeitweise wird in der Sensor-Fluidführungseinheit und / oder der Sensor-Anordnung ein Unterdruck relativ zu einer Umgebung des Messsystems hergestellt.
- Eine Gasprobe wird aus der Patienten-Fluidführungseinheit abgezweigt und durch die Sensor-Fluidführungseinheit hindurch zur Sensor-Anordnung geleitet. Hierzu trägt der Unterdruck bei. Möglich ist, dass die Gasprobe aus der Patienten-Fluidführungseinheit abgesaugt wird, wofür ein Unterdruck relativ zur Patienten-Fluidführungseinheit hergestellt wird. Möglich ist auch, dass zusätzlich ein Überdruck in der Patienten-Fluidführungseinheit relativ zur Umgebung bewirkt, dass die Gasprobe abgezweigt wird.
- Ermittelt wird ein zeitlicher Verlauf der Wärmeleitfähigkeit der Gasprobe, welche abgezweigt wurde und die Sensor-Anordnung erreicht. Dieser zeitliche Verlauf wird unter Verwendung von Messwerten des Wärmeleitfähigkeits-Sensors ermittelt und als "Wärmeleitfähigkeits-Verlauf" bezeichnet.
- Entschieden wird, ob ein Indiz für ein Leck zwischen der Patienten-Fluidführungseinheit und der Sensor-Anordnung aufgetreten ist. Diese Entscheidung wird abhängig von einer zeitlichen Veränderung (Ableitung nach der Zeit) der ermittelten Wärmeleitfähigkeit automatisch getroffen, und zwar von der Signalverarbeitungseinheit. Optional wird für die Entscheidung mindestens ein weiteres Signal verwendet.
- Erfindungsgemäß wird also ein Indiz für ein Leck detektiert, oder entschieden wird, dass aktuell kein solches Indiz vorliegt. Das Leck, für das erfindungsgemäß ein Indiz detektiert oder ausgeschlossen wird, stellt wenigstens zeitweise eine Fluidverbindung zwischen der Sensor-Fluidführungseinheit und / oder der Sensor-Anordnung einerseits und der Umgebung des Messsystems andererseits her.

Unter einer "Fluidführungseinheit" wird ein Bauteil verstanden, welches ein Fluid entlang einer Trajektorie leitet, wobei diese Trajektorie durch die Geometrie, Konstruktion und Anordnung des Bauteils vorgegeben ist. Ein Faltenschlauch, ein glatter Schlauch und eine Röhre sind Beispiele für eine Fluidführungseinheit. Die Fluidführungseinheit umfasst nicht notwendigerweise eine Fördereinheit.

In vielen Fällen ist es für eine zuverlässige künstliche Beatmung des Patienten erforderlich, dass der zeitliche Verlauf der Konzentration mindestens eines Bestandteils des Gasgemischs, welches durch die Patienten-Fluidführungseinheit fließt, ausreichend genau gemessen wird. Die Konzentration ist insbesondere der Anteil in Vol.-%. Beispielsweise soll der tatsächliche zeitliche Verlauf einem vorgegebenen zeitlichen Sollverlauf folgen. Das medizinische Gerät wird entsprechend geregelt, wofür der tatsächliche zeitliche Konzentrationsverlauf zuverlässig gemessen werden muss. Die Sensor-Anordnung ist dazu ausgestaltet, ein Maß für die aktuelle Konzentration des Bestandteils zu messen. Möglich ist, dass die Sensor-Anordnung jeweils ein Maß für die tatsächliche Konzentration von mindestens zwei Bestandteilen der Gasprobe zu messen vermag.

DE 10 2020 002570 A1 offenbart ein Verfahren und eine Vorrichtung, um während einer künstlichen Beatmung eines Patienten ein Leck zu erkennen, wobei dieses Leck die Messung von Gaskonzentrationen, die im Beatmungskreislauf auftreten, beeinflussen kann.

DE 10 2010 014883 A1 und DE 10 2020 117607 A1 offenbaren Vorrichtungen zur Messung der Gaskonzentration in einer Gasprobe durch Messung der Wärmeleitfähigkeit der Gasprobe.

Erfindungsgemäß wird eine Gasprobe aus der Patienten-Fluidführungseinheit abgezweigt, zur Sensor-Anordnung geleitet und von der Sensor-Anordnung untersucht. Das Volumen der abgezweigten und untersuchten Gasprobe ist im Allgemeinen klein im Vergleich zum Volumen des Fluids, welches sich zu einem Zeitpunkt in der Patienten-Fluidführungseinheit befindet. Daher nehmen das erfindungsgemäße Überwachungs-Verfahren und das erfindungsgemäße Messsystem in der Regel keinen relevanten Einfluss auf eine künstliche Beatmung des Patienten. Bevorzugt wird die abgezweigte Gasprobe durch die Sensor-Anordnung geleitet und anschließend wieder in die Patienten-Fluidführungseinheit eingespeist.

Ein Leck kann ein Messergebnis der Sensor-Anordnung verfälschen. Durch dieses Leck hindurch kann nämlich aufgrund des Unterdrucks relativ zur Umgebung Umgebungsluft eindringen und zur Sensor-Anordnung gelangen, so dass die Sensor-Anordnung nicht das Gasgemisch aus der Patienten-Fluidführungseinheit analysiert, sondern eine in der Regel nicht bekannte Zusammensetzung aus diesem Gasgemisch und der Umgebungsluft. Dies kann zu einem falschen Messergebnis und daher zu einer fehlerhaften künstlichen Beatmung führen. Insbesondere kann ein zu niedriger Anteil an Sauerstoff gemessen werden, wenn das Gasgemisch einen höheren Sauerstoffanteil als die Umgebungsluft aufweist. Daher ist es wichtig, zuverlässig und rasch ein Indiz für ein Leck zu detektieren. Falls ein solches Indiz detektiert ist, lässt sich das Messsystem automatisch und / oder manuell genauer überprüfen, und das Leck lässt sich anschließend beseitigen - oder es wird ausgeschlossen, dass tatsächlich ein Leck aufgetreten ist.

Erfindungsgemäß wird der Wärmeleitfähigkeits-Verlauf, also der zeitliche Verlauf der Wärmeleitfähigkeit der Gasprobe, ermittelt. Für diese Ermittlung wird die Wärmeleitfähigkeit an einer Messposition in der Sensor-Anordnung oder flussaufwärts von der Sensor-Anordnung und flussabwärts von einem möglichen zu detektierenden Leck verwendet. Die Entscheidung, ob ein Indiz für ein Leck vorliegt oder nicht, wird in Abhängigkeit von der zeitlichen Veränderung (Ableitung nach der Zeit) der ermittelten Wärmeleitfähigkeit gefällt.

Möglich ist, die Entscheidung über das Vorhandensein eines Lecks zusätzlich abhängig vom gemessenen zeitlichen Verlauf der Konzentration eines Bestandteils in der abgezweigten Gasprobe und / oder vom gemessenen Druck-Verlauf der Gasprobe zu fällen. Erfindungsgemäß wird der Wärmeleitfähigkeits-Verlauf zusätzlich zu oder anstelle eines zeitlichen Verlaufs der Konzentration eines Bestandteils der Gasprobe und / oder des Drucks der Gasprobe verwendet. Dieses Merkmal hat insbesondere den folgenden Vorteil: In der Regel wird durch die Patienten-Fluidführungseinheit ein Gasgemisch mit unterschiedlichen Bestandteilen geleitet. Einige dieser Bestandteile, insbesondere Sauerstoff und Kohlendioxid, kommen auch in der Luft und daher auch in der Umgebung der Patienten-Fluidführungseinheit und in der Umgebung des Messsystems vor. Möglich ist, dass die Konzentration eines Bestandteils des Gasgemischs in der Patienten-Fluidführungseinheit nur wenig von der Konzentration dieses Bestandteils in der Umgebung abweicht. Beispielsweise wird manchmal eine lungenprotektive künstliche Beatmung eingesetzt, bei welcher die Konzentration von Sauerstoff in dem zugeführten Gasgemisch nur relativ wenig oberhalb der Konzentration von Sauerstoff in der Luft liegt. Ein Leck verändert daher die Konzentration von Sauerstoff in der Sensor-Fluidführungseinheit und in der Sensor-Anordnung nur relativ wenig, verglichen mit einem Zustand ohne ein Leck. In diesem Falle ist die Konzentration von Sauerstoff in der Gasprobe allein kein ausreichend zuverlässiger Indikator, um ein Leck zu detektieren. In vielen Fällen wirkt sich ein relativ kleines Leck auch nicht stark auf den Druck in der Patienten-Fluidführungseinheit aus. In diesem Fall ist auch der Druck allein kein zuverlässiger Indikator. Falls die Detektion eines Lecks nur darauf beruht, dass die Konzentration mindestens eines Gasproben-Bestandteils, der auch in der Umgebungsluft vorkommt, und / oder der Druck gemessen wird, so ist die Gefahr größer als beim erfindungsgemäßen Überwachungs-Verfahren und beim erfindungsgemäßen Messsystem, dass ein Leck nicht detektiert wird.

Ein weiterer Vorteil der Erfindung ist der folgende: Falls die Detektion eines Lecks ausschließlich vom jeweiligen zeitlichen Verlauf der Konzentration mindestens eines Bestandteils der Gasprobe abhängen würde, so kann die Zuverlässigkeit der Detektion stark davon abhängen, welche Bestandteile der Gasprobe für die Überwachung gewählt werden. Entweder lässt sich in diesem Fall ein derartiges Überwachungs-Verfahren nur für bestimmte Situationen verwenden. Oder es muss vorab gemessen werden, welche Bestandteile im Gasgemisch in der Patienten-Fluidführungseinheit vorkommen und ob deren jeweilige Konzentration stark von der Konzentration in der Umgebungsluft abweicht. Die Erfindung spart eine solche Notwendigkeit und eine solche Restriktion ein.

Der Druck im Messsystem hängt in aller Regel wesentlich stärker von dem zeitlich variierenden Druck, der in der Patienten-Fluidführungseinheit herrscht, ab als von der Druckveränderung aufgrund eines Lecks. Ein Grund: Häufig werden bei Atmungsschübe ausgeführt. Daher ist in vielen Fällen der Druck auch kein geeigneter Indikator, um ein Leck zuverlässig zu detektieren.

Das Gasgemisch, das durch die Patienten-Fluidführungseinheit hindurch geleitet wird, enthält in der Regel auch Bestandteile, die überhaupt nicht oder nur mit einer wesentlich geringeren oder auch höheren Konzentration in der Umgebung auftreten. Insbesondere tritt in der Regel Stickstoff in der Umgebungsluft mit einer wesentlich höheren Konzentration auf als in dem Gasgemisch in der Patienten-Fluidführungseinheit. Hingegen treten in der Regel in der Umgebung weder ein Narkosemittel noch Lachgas mit einer relevanten Konzentration auf. Die üblicherweise verwendeten Narkosemittel sowie Lachgas haben eine deutlich geringere Wärmeleitfähigkeit als Luft. Daher beeinflusst ein Leck in der Regel signifikant die Wärmeleitfähigkeit der Gasprobe in der Sensor-Anordnung. Eine veränderte Wärmeleitfähigkeit ist also in der Regel ein zuverlässiger Indikator dafür, dass ein Leck aufgetreten ist.

Auch dann, wenn kein Leck vorhanden ist, variiert in der Regel die Wärmeleitfähigkeit des Gasgemischs in der Patienten-Fluidführungseinheit und damit in der abgezweigten Gasprobe über die Zeit. Dies gilt insbesondere dann, wenn als medizinisches Gerät ein Beatmungsgerät verwendet wird, welches eine Abfolge von Beatmungshüben durchführt und in jedem Beratungshub jeweils eine Menge des Gasgemischs zur patientenseitigen Koppeleinheit fördert. Typischerweise oszilliert die Wärmeleitfähigkeit in Abhängigkeit von diesen Beatmungshüben. Daher wird erfindungsgemäß eine zeitliche Veränderung der Wärmeleitfähigkeit verwendet und nicht lediglich ein momentaner Wert. Dies erhöht weiter die Zuverlässigkeit, dass tatsächlich ein Leck detektiert wird, und reduziert die Gefahr von Fehlalarmen. Insbesondere wird die Zuverlässigkeit vergrößert, dass die Oszillation der Wärmeleitfähigkeit aufgrund eines Lecks von einer Oszillation aufgrund von Beatmungshüben unterschieden wird, insbesondere wenn ein Glättungsverfahren auf den Wärmeleitfähigkeits-Verlauf angewendet wird. Ein Sonderfall des Wärmeleitfähigkeits-Verlaufs ist, dass die Wärmeleitfähigkeit oder ein gleitender Durchschnitt der Wärmeleitfähigkeit idealerweise konstant bleibt, solange kein Leck aufgetreten ist. Die zeitliche Veränderung der Wärmeleitfähigkeit ist dann Null.

Die Wärmeleitfähigkeit des Gasgemischs in der Patienten-Fluidführungseinheit kann auch durch andere Ereignisse verändert werden. Häufig werden wenigstens einige dieser Ereignisse bewusst herbeigeführt, beispielsweise weil das medizinische Gerät als Reaktion auf eine Benutzervorgabe oder eine Vorgabe einer übergeordneten Steuerung die Konzentration eines Bestandteils des Gasgemischs in der Patienten-Fluidführungseinheit stark vergrößert oder auch verkleinert. Beispielsweise wird die Konzentration von Sauerstoff gewollt schlagartig vergrößert. Ein solches Ereignis lässt sich detektieren oder erfassen, beispielsweise indem eine entsprechende Nachricht von einem Steuergerät des medizinischen Geräts erfasst und bei der Entscheidung, ob ein Indiz für ein Leck vorliegt oder nicht, berücksichtigt wird.

Sowohl bei bekannten Verfahren und Vorrichtungen zur Leck-Detektion als auch bei dem erfindungsgemäßen Überwachungs-Verfahren und dem erfindungsgemäßen Messsystem kann ein Fehlalarm erzeugt werden - genauer gesagt: Ein Indiz für ein Leck wird detektiert, obwohl in Wirklichkeit kein Leck aufgetreten ist. Zum einen ist es in der Regel wichtig, jedes Leck zuverlässig zu detektieren, auch wenn dann Fehlalarme ausgelöst werden. Zum anderen lässt sich in vielen Fällen nach der Detektion des Indizes durch eine genauere Prüfung überprüfen, ob tatsächlich ein Leck aufgetreten ist oder nicht.

In der Regel bewirkt ein zu detektierendes Leck, dass ein Volumenfluss durch das Leck hindurch auftritt, und zwar aufgrund des Unterdrucks ein Volumenfluss von der Umgebung zu der Sensor-Anordnung. In einer bevorzugten Ausgestaltung wird wiederholt ein erstes Maß für einen relativen Leck-Volumenfluss ermittelt. Unter einem "Volumenfluss" wird wie üblich das Volumen pro Zeiteinheit eines Fluids, das durch eine Fluidführungseinheit oder auch durch ein Leck fließt, verstanden. Der relative Leck-Volumenfluss gemäß der Ausgestaltung der Erfindung ist der Anteil des Volumenflusses durch das Leck an dem gesamten Volumenfluss zur Sensor-Anordnung. Der Volumenfluss zur Sensor-Anordnung ist die Summe aus

- dem Volumenfluss, mit dem die Gasprobe aus der Patienten-Fluidführungseinheit abgezweigt wird und durch die Sensor-Fluidführungseinheit zur Sensor-Anordnung fließt, und
- dem Volumenfluss durch das Leck hindurch.

In der Regel ist das erste Maß umso größer, je größer der relative Leck-Volumenfluss ist. In vielen Fällen bleiben der gesamte Volumenfluss zur Sensor-Anordnung und damit auch diese Summe zeitlich konstant, insbesondere wenn die Gasprobe unter Verwendung einer Pumpe oder sonstigen Fluidfördereinheit mit konstanter Förderleistung abgezweigt wird. Jedoch ändert sich durch ein Leck der relative Leck-Volumenfluss.

Gemäß einer bevorzugten Ausgestaltung wird das erste Maß für den relativen Leck-Volumenfluss abhängig von dem Wärmeleitfähigkeits-Verlauf, den die Sensor-Anordnung gemessen hat, ermittelt. Außerdem wird die Wärmeleitfähigkeit der Umgebung (idR. der Umgebungsluft) vorgegeben und verwendet. Die Wärmeleitfähigkeit von Umgebungsluft ist in der Regel bekannt und wird vorgegeben. Möglich ist auch, die Wärmeleitfähigkeit der Umgebung durch folgendes Verfahren zu messen: Für einen kurzen Messzeitraum wird eine Fluidverbindung zwischen der Sensor-Fluidführungseinheit und der Umgebung hergestellt, also bewusst ein Leck erzeugt. In einer Ausgestaltung wird zugleich die Fluidverbindung zwischen der Sensor-Fluidführungseinheit und der Patienten-Fluidführungseinheit unterbrochen. Dadurch wird im Messzeitraum ausschließlich Umgebungsluft zur Sensor-Anordnung gesaugt. Im Messzeitraum misst der Wärmeleitfähigkeits-Sensor der Sensor-Anordnung die Wärmeleitfähigkeit der Umgebung, z.B. der Umgebungsluft, und nicht die des Gasgemischs in der Patienten-Fluidführungseinheit.

Häufig ist auch der gesamte Volumenfluss zur Sensor-Anordnung bekannt, insbesondere wenn eine Pumpe oder sonstige Fluidfördereinheit die Gasprobe ansaugt und der Volumenfluss, den die Fluidfördereinheit erzeugt, bekannt ist, insbesondere zeitlich konstant ist.

Die Entscheidung, ob ein Indiz für ein Leck aufgetreten ist oder nicht, wird in Abhängigkeit von einer zeitlichen Veränderung (Ableitung nach der Zeit) dieses ersten Maßes für den relativen Leck-Volumenfluss, das auf der Wärmeleitfähigkeit beruht, gefällt.

Die Verwendung eines Maßes für einen relativen Leck-Volumenfluss hat folgenden Vorteil gegenüber anderen möglichen Vorgehensweisen, um die Wärmeleitfähigkeit für die Detektion eines Indizes für ein Leck zu verwenden: Falls ein Leck aufgetreten ist, so vergrößert sich der relative Leck-Volumenfluss. Falls das erste Maß so festgelegt ist, dass das erste Maß umso größer ist, je größer der relative Leck-Volumenfluss ist, so führt ein Leck zu einem ansteigenden ersten Maß, ansonsten zu einem abnehmenden ersten Maß. Diese Auswirkung gilt unabhängig davon, ob das Leck die Wärmeleitfähigkeit der Gasprobe, die zur Sensor-Anordnung fließt, vergrößert oder verkleinert, verglichen mit einem Zustand ohne Leck. Mit anderen Worten: Diese Auswirkung gilt unabhängig davon, ob die Luft oder das sonstige Gasgemisch in der Umgebung des Messsystems eine größere oder eine kleinere Wärmeleitfähigkeit als die abgezweigte Gasprobe aufweist. Daher erspart diese Ausgestaltung die Notwendigkeit, vorzugeben oder zu messen, ob ein Leck die Wärmeleitfähigkeit vergrößert oder verkleinert.

Die Entscheidung, ob ein Indiz für ein Leck aufgetreten ist, wird in Abhängigkeit mindestens von einer zeitlichen Veränderung desjenigen Maßes für den relativen Leck-Volumenfluss getroffen, das von der Wärmeleitfähigkeit abhängt.

Bevorzugt wird nur dann ein Indiz für ein Leck erkannt, wenn das erste Maß ausreichend stark und ausreichend lange von einem Referenz-Zustand abweicht. Eine untere Zeitdauer-Schranke und eine untere Veränderungs-Schranke werden vorgegeben. Ein Indiz für ein Leck ist erkannt, wenn folgende Bedingungen kumulativ erfüllt sind:

- Das erste Maß in einem Entscheidungs-Zeitraum weicht vom ersten Maß in einem Referenz-Zeitraum ab.
- Der Referenz-Zeitraum liegt vor dem Entscheidungs-Zeitraum.
- Sowohl der Referenz-Zeitraum als auch der Entscheidungs-Zeitraum haben eine Dauer, die mindestens so lang wie die untere Zeitdauer-Schranke ist.
- Die Abweichung zwischen dem ersten Maß im Entscheidungs-Zeitraum und dem ersten Maß im Referenz-Zeitraum ist mindestens so groß wie die untere Veränderungs-Schranke.

Bevorzugt misst der Wärmeleitfähigkeits-Sensor laufend die Wärmeleitfähigkeit der Gasprobe, und der Entscheidungs-Zeitraum ist ein gleitender Zeitraum, der beispielsweise im aktuellen Zeitpunkt endet, bevorzugt ein Zeitraum von fester Zeitdauer.

In einer Ausgestaltung ist das erste Maß ein Schätzwert für den relativen Leck-Volumenfluss, wofür eine vorgegebene Rechenvorschrift angewendet wird. Beispielsweise wird das erste Maß vorab unter Verwendung der folgenden Annahme aufgestellt: Die Wärmeleitfähigkeit des Gasgemischs, welches die Sensor-Anordnung erreicht, ist ein gewichtetes Mittel aus der Wärmeleitfähigkeit der abgezweigten Gasprobe und der Wärmeleitfähigkeit des Gases in der Umgebung des Messsystems, in der Regel der Umgebungsluft. Der Gewichtsfaktor, mit dem das Gas aus der Umgebung in die Wärmeleitfähigkeit des Gasgemischs eingeht, ist der relative Leck-Volumenfluss oder hängt vom relativen Leck-Volumenfluss ab. Falls kein Leck vorliegt, so ist dieser Gewichtsfaktor idealerweise gleich Null. Die Wärmeleitfähigkeit des Gases in der Umgebung wird vorgegeben oder gemessen, z.B. so wie weiter oben beschrieben. Die Wärmeleitfähigkeit des Gasgemischs, welches die Sensor-Anordnung erreicht, wird erfindungsgemäß vom Wärmeleitfähigkeits-Sensor der Sensor-Anordnung gemessen. Die Wärmeleitfähigkeit der abgezweigten Gasprobe wird in einer Ausgestaltung zu einem Zeitpunkt gemessen, an dem feststeht, dass kein Leck aufgetreten ist, oder im medizinischen Gerät oder in der Patienten-Fluidführungseinheit gemessen. Daher ist der relative Leck-Volumenfluss die einzige Unbekannte in diesem Zusammenhang. Das erste Maß ist umso größer, je größer der relative Leck-Volumenfluss ist, und ist in der Regel nur eine Näherung für den tatsächlichen relativen Leck-Volumenfluss.

Erfindungsgemäß wird die Entscheidung, ob ein Indiz für ein Leck aufgetreten ist, abhängig von dem gemessenen Wärmeleitfähigkeits-Verlauf der Gasprobe, die die Sensor-Anordnung erreicht, getroffen. Bevorzugt wird für diese Entscheidung ein erstes Maß für den relativen Leck-Volumenfluss verwendet, wobei das erste Maß auf der Wärmeleitfähigkeit beruht, beispielsweise gemäß der gerade genannten Rechenvorschrift. Gemäß einer Ausgestaltung wird die zeitliche Veränderung des ersten Maßes für die Entscheidung verwendet.

Bevorzugt wird die Entscheidung, ob ein Indiz für ein Leck aufgetreten ist oder nicht, zusätzlich in Abhängigkeit von mindestens einer weiteren Messgröße getroffen. In einer ersten Alternative ist die oder mindestens eine weitere Messgröße der zeitliche Verlauf der Konzentration eines Bestandteils der Gasprobe. Dieser Bestandteil ist beispielsweise Sauerstoff oder Kohlendioxid oder Lachgas oder ein Anästhesiemittel (Narkosemittel). Möglich ist, zwei verschiedene Messgrößen zu verwenden, wobei jede Messgröße auf dem zeitlichen Verlauf jeweils eines Bestandteils der Gasprobe beruht und die beiden Messgrößen sich auf unterschiedliche Bestandteile beziehen. Möglich ist auch, dass die oder mindestens eine Messgröße der zeitliche Verlauf der summierten Konzentrationen mehrerer Bestandteile ist. In einer zweiten Alternative ist die weitere Messgröße der zeitliche Verlauf des Drucks der Gasprobe. Diese beiden Alternativen lassen sich miteinander kombinieren, so dass die Wärmeleitfähigkeit und mindestens zwei weitere Messgrößen verwendet werden.

In einer Realisierungsform wird abhängig von dem Konzentrations-Verlauf und / oder dem Druck-Verlauf ein zweites Maß für den relativen Leck-Volumenfluss berechnet. Für die Entscheidung, ob ein Indiz für ein Leck aufgetreten ist, wird von einer zeitlichen Veränderung des ersten Maßes und einer zeitlichen Veränderung des zweiten Maßes gefällt.

In einer ersten Alternative wird dann entschieden, dass ein Indiz für ein Leck vorliegt, wenn sowohl die Wärmeleitfähigkeit als auch die oder mindestens eine weitere Messgröße sich ändern, und zwar - bis auf eine Toleranz - zum gleichen Zeitpunkt und bevorzugt für mindestens eine untere Zeitdauer-Schranke. Bei dieser Alternative ist die Gefahr geringer, dass ein Fehlalarm ausgelöst wird. Denn eine veränderte Wärmeleitfähigkeit allein wird in manchen Situationen nicht von einem Leck hervorgerufen, sondern von einem gewollt herbeigeführten Ereignis, beispielsweise durch eine gewollte Veränderung des Gasgemischs, welches das medizinische Gerät bei der künstlichen Beatmung bereitstellt.

In einer zweiten Alternative wird dann entschieden, dass ein Indiz für ein Leck vorliegt, wenn die Wärmeleitfähigkeit oder die oder mindestens eine weitere Messgröße sich ändern, aber nicht notwendigerweise sowohl die Wärmeleitfähigkeit als auch die weitere Messgröße. Diese zweite Alternative steigert die Sicherheit, dass tatsächlich jedes Leck detektiert wird.

Eine bevorzugte Ausgestaltung des erfindungsgemäßen Messsystems stellt ein Messverfahren und einen Sensor bereit, die sowohl die Wärmeleitfähigkeit als auch die Konzentration eines Bestandteils der Gasprobe zu messen vermögen. Voraussetzung ist, dass der Bestandteil ein paramagnetisches Gas ist und mit einer ausreichend großen Konzentration in der Gasprobe enthalten ist. Sauerstoff ist ein paramagnetisches Gas, das in aller Regel in dem Gasgemisch enthalten ist, welches durch die Patienten-Fluidführungseinheit hindurch zur patientenseitigen Koppeleinheit geleitet wird, und dessen Konzentration in vielen Fällen ohnehin gemessen werden soll.

Gemäß der Ausgestaltung werden folgende Schritte durchgeführt, und die Sensor-Anordnung ist dazu ausgestaltet, die folgenden Schritte durchzuführen:

- Die Gasprobe oder wenigstens ein Teil der Gasprobe wird in eine Messkammer geleitet.
- An die Messkammer wird ein Magnetfeld angelegt. Dieses Magnetfeld wird dergestalt angelegt, dass es eine oszillierende Feldstärke aufweist.
- Ein Heizelement wird erhitzt. Das erhitzte Heizelement führt der Gasprobe in der Messkammer Wärmeenergie zu.
- Eine elektrische Detektionsgröße des Heizelements wird gemessen. Die Detektionsgröße korreliert mit der Wärmeleitfähigkeit der Gasprobe in der Messkammer und ist insbesondere die elektrische Leistung, welche das Heizelement aufnimmt, oder die elektrische Spannung, die am Heizelement anliegt.
- Weil das Magnetfeld eine oszillierende Feldstärke aufweist, oszilliert auch die gemessene elektrische Detektionsgröße, und zwar synchron mit oder wenigstens abhängig von der Oszillation der Magnetfeldstärke.
- Ein oszillierendes Signal und ein weiteres Signal werden hergeleitet. Das oszillierende Signal oszilliert abhängig von der, insbesondere synchron mit der Magnetfeldstärke. Der zeitliche Verlauf des weiteren Signals hängt nicht von der Oszillation der Magnetfeldstärke ab. Um die beiden Signale herzuleiten, wird auf die elektrische Detektionsgröße des Heizelements eine Filterung angewendet.
- Das oszillierende Signal korreliert mit dem zeitlichen Verlauf der Wärmeleitfähigkeit des parametrischen Gases in der Gasprobe und wird als Maß für die zeitlich veränderliche Konzentration des parametrischen Gases verwendet.
- Das weitere Signal korreliert mit dem Wärmeleitfähigkeits-Verlauf der Gasprobe und wird als ein Maß für die zeitlich veränderliche Wärmeleitfähigkeit der Gasprobe verwendet. Die Oszillation der Feldstärke des angelegten Magnetfelds beeinflusst nämlich in der Regel die Wärmeleitfähigkeit der Gasprobe nicht in nennenswertem Umfang.

Verglichen mit einer Ausgestaltung, bei der ein erster Sensor für die Wärmeleitfähigkeit und ein zweiter Sensor für die Konzentration verwendet wird, spart diese Ausgestaltung Platz und Bauteile ein. Denn mehrere Elemente der Sensor-Anordnung lassen sich sowohl dafür verwenden, die Wärmeleitfähigkeit der Gasprobe zu messen, als auch dafür, die Konzentration des paramagnetischen Gases zu messen. Falls in der Gasprobe mindestens zwei verschiedene paramagnetische Gase enthalten sind, so wird durch diese Ausgestaltung in der Regel die Summe der Konzentrationen beider Gase gemessen.

In einer Ausgestaltung löst der Schritt, dass erfindungsgemäß ein Indiz für ein Leck detektiert wurde, den Schritt aus, dass ein Alarm in mindestens einer von einem Menschen wahrnehmbaren Form ausgegeben wird. In einer bevorzugten Ausgestaltung wird hingegen zunächst eine Überprüfungs-Abfolge ausgelöst, um zu überprüfen, ob tatsächlich ein Leck aufgetreten ist oder nicht. Der Alarm wird ausgegeben, wenn die Überprüfungs-Abfolge mit ausreichender Zuverlässigkeit das Ergebnis liefert, dass tatsächlich ein Leck aufgetreten ist. Die Überprüfungs-Abfolge umfasst die folgenden Schritte, die automatisch durchgeführt werden:

- Der Schritt, dass die Gasprobe abgezweigt und durch die Sensor-Fluidführungseinheit hindurch zu Sensor-Anordnung geleitet wird, wird im Überprüfungs-Zeitraum unterbrochen. Die Sensor-Fluidführungseinheit steht also im Überprüfungs-Zeitraum nicht in einer Fluidverbindung mit der Patienten-Fluidführungseinheit.
- Ein Maß für den Druck in der Sensor-Anordnung und / oder in der Sensor-Fluidführungseinheit wird gemessen.
- Ein Maß für den Druck in der Patienten-Fluidführungseinheit wird gemessen.
- Die beiden gemessenen Drücke werden miteinander verglichen.
- Wenn die Abweichung zwischen den beiden gemessenen Drücken ein vorgegebenes Kriterium erfüllt, wird entschieden, dass ein Leck vorliegt. Bevorzugt wird insbesondere dann entschieden, dass ein Leck vorliegt, wenn die Abweichungen zwischen den beiden Drücken in einem Entscheidungs-Zeitraum um stärker als eine vorgegebene Mindestschranke voneinander abweichen.

Im Überprüfungs-Zeitraum kann die Sensor-Anordnung nicht die Konzentration eines Bestandteils des Gasgemischs, welche durch die Patienten-Fluidführungseinheit fließt, messen. Dank der Erfindung braucht die Überprüfungs-Abfolge nur dann durchgeführt zu werden, wenn ein Indiz für ein Leck detektiert wurde. Bevorzugt wird hingegen im laufenden Betrieb überprüft, ob das Indiz für das Leck aufgetreten ist, also während die Sensor-Anordnung die Konzentration mindestens eines Bestandteils misst.

Die Erfindung betrifft weiterhin eine Beatmungs-Anordnung, welche einen Patienten künstlich zu beatmen vermag. Ein entsprechendes Beatmungsverfahren wird auch beschrieben, jedoch nicht beansprucht. Der Patient ist mit einer patientenseitigen Koppeleinheit verbunden oder lässt sich wenigstens zeitweise mit einer patientenseitigen Koppeleinheit verbinden. Das Beatmungs-Verfahren umfasst den Schritt, dass ein Gasgemisch von einem medizinischen Gerät durch eine Patienten-Fluidführungseinheit hindurch zur patientenseitigen Koppeleinheit gefördert wird. Das Gasgemisch umfasst Sauerstoff und optional mindestens ein Narkosemittel. Die Beatmungs-Anordnung umfasst ein medizinisches Gerät, eine Patienten-Fluidführungseinheit und die patientenseitige Koppeleinheit und ist dazu ausgestaltet, ein Gasgemisch vom medizinischen Gerät zur patientenseitigen Koppeleinheit zu fördern.

Das Beatmungs-Verfahren wird unter Verwendung eines erfindungsgemäßen Messsystems durchgeführt, und die Beatmungs-Anordnung umfasst zusätzlich ein erfindungsgemäßes Messsystem. Durch ein erfindungsgemäßes Überwachungs-Verfahren wird entschieden, ob ein Indiz für ein Leck zwischen der Patienten-Fluidführungseinheit und der Sensor-Anordnung des Messsystems aufgetreten ist. Die Beatmungs-Anordnung vermag eine solche Entscheidung automatisch zu treffen.

Vorteilhafte Ausgestaltungen des erfindungsgemäßen Überprüfungs-Verfahrens sind auch vorteilhafte Ausgestaltungen des Beatmungs-Verfahrens. Vorteilhafte Ausgestaltungen des erfindungsgemäßen Messsystems sind auch vorteilhafte Ausgestaltungen der Beatmungs-Anordnung.

Im Folgenden wird die Erfindung anhand eines Ausführungsbeispiels beschrieben. Hierbei zeigt

Figur 1   schematisch einen Beatmungskreislauf zur künstlichen Beatmung eines Patienten;

Figur 2   schematisch eine erste Ausführungsform eines Sensors, welcher sowohl den Sauerstoffgehalt als auch die Wärmeleitfähigkeit der abgezweigten Gasprobe misst;

Figur 3   schematisch eine zweite Ausführungsform eines solchen Sensors;

Figur 4   die Messeinheit des Sensors von Figur 3;

Figur 5   eine beispielhafte Auswerteschaltung des Sensors von Figur 3;

Figur 6   beispielhaft den zeitlichen Verlauf der Sauerstoff-Konzentration und der $CO_2$-Konzentrationin der abgezweigten Gasprobe;

Figur 7   beispielhaft den zeitlichen Verlauf der drei Maße für den relativen Leck-Volumenfluss.

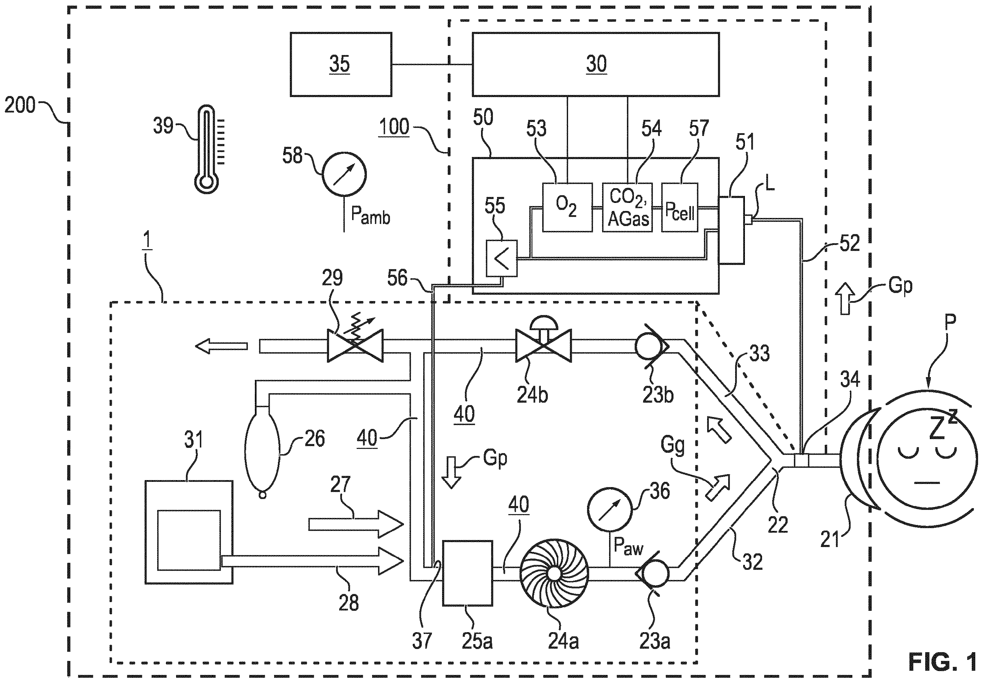

Figur 1 zeigt schematisch eine Beatmungs-Anordnung 200 mit einem schematisch gezeigten Beatmungsgerät 1, wobei die Beatmungs-Anordnung 200 einen Patienten P künstlich zu beatmen vermag. Vom Patienten P wird lediglich dessen Gesicht schematisch gezeigt. Das Beatmungsgerät 1 hält einen Beatmungskreislauf 40 aufrecht. Im Ausführungsbeispiel ist das Beatmungsgerät 1 als Anästhesiegerät ausgestaltet, und der Patient P ist narkotisiert oder wenigstens sediert.

Im Beatmungskreislauf 40 wird dem Patienten P ein Gasgemisch Gg zugeführt, welches Sauerstoff ($O_2$), optional Kohlendioxid ($CO_2$), im Ausführungsbeispiel mindestens ein Narkosemittel, optional ein Trägergas für das Narkosemittel und optional weitere Bestandteile umfasst. Die vom Patienten P ausgeatmete Luft gelangt bevorzugt wieder in den Beatmungskreislauf 40 und daher nicht in der Umgebung.

Die Erfindung lässt sich auch für eine künstliche Beatmung einsetzen, bei der dem Patient P zwar ein Gasgemisch Gg umfassend Sauerstoff zugeführt wird, dieses Gasgemisch Gg aber kein Narkosemittel umfasst. Die vom Patienten P ausgeatmeten Luft darf daher in die Umgebung gelangen. Daher wird bevorzugt keine Beatmungskreislauf realisiert, sondern lediglich eine Patienten-Fluidverbindung vom Beatmungsgerät 1 zum Patienten P.

Möglich ist, dass die künstliche Beatmung mit oder ohne Narkosemittel und die eigene Atmungsaktivität des Patienten P sich überlagern. Die eigene Atmungsaktivität des Patienten P wird von dessen Atmungsmuskulatur bewirkt, und zwar durch seine spontane Atmung und optional durch eine externe Stimulation der Atmungsmuskulatur.

Eine nur schematisch gezeigte patientenseitige Koppeleinheit 21, beispielsweise ein Mundstück oder eine Atemmaske oder ein Tubus, verbindet den Patienten P mit dem Beatmungskreislauf 40 oder mit der Patienten-Fluidverbindung. Die patientenseitige Koppeleinheit 21 ist mit einem Y-Stück 22 verbunden. Das Y-Stück 22 ist mit einer Atemgasleitung 32 für die Einatmung (Inspiration) und mit einer Atemgasleitung 33 für die Ausatmung (Exspiration) verbunden.

Eine Pumpe 24a saugt Atemgas an und erzeugt einen permanenten Strom von Atemluft durch die Einatmungs-Atemgasleitung 32 auf das Y-Stück 22 und die patientenseitigen Koppeleinheit 21 und damit auf den Patienten P zu. Bevorzugt arbeitet diese Pumpe 24a als ein Verdichter, der einen Überdruck erzeugt und mit einer Drehzahl von über 10.000 Umdrehungen/min rotiert. Ein Kohlendioxidabsorber ($CO_2$-Absorber) 25a vermag Kohlendioxid ($CO_2$) aus dem Beatmungskreislauf 40 zu absorbieren. Ein Rückschlagventil (check valve, nonreturn valve) 23a lässt einen Gasfluss in der Einatmungs-Atemgasleitung 32 auf das Y-Stück 22 zu durch und sperrt einen Gasfluss in die umgekehrte Richtung.

Ein Rückschlagventil 23b lässt einen Gasfluss in der Ausatmungs-Atemgasleitung 33 vom Y-Stück 22 weg passieren und sperrt einen Gasfluss in die umgekehrte Richtung.

Ein ansteuerbares PEEP-Ventil 24b (PEEP = "positive end-expiratory pressure") lässt je nach seiner Stellung den Luftstrom, den die Pumpe 24a erzeugt hat, passieren oder sperrt ihn, trägt dadurch zur Erzeugung der einzelnen Beatmungshübe bei und legt die Amplituden und Frequenzen dieser Beatmungshübe fest. Das PEEP-Ventil 24b stellt außerdem sicher, dass auch am Ende der Ausatmung oder bei einer kurzfristigen Öffnung oder Unterbrechung des Beatmungskreislaufs 40 ein ausreichend großer Luftdruck in der Lunge des Patienten P aufrecht erhalten bleibt. Dadurch wird die Gefahr verringert, dass die Lunge des Patienten P durch einen zu geringen Druck kollabiert.

Ein Überdruckventil 29 vermag einen Überdruck im Beatmungskreislauf 40 abzubauen, indem Atemgas abgegeben wird, und zwar bevorzugt in eine Fortleitung für Narkosegas oder auch in die Umgebung. Dieses Überdruckventil 29 ist bevorzugt als "adjustable pressure limiting valve" ausgestaltet und reduziert die Gefahr, dass die Lunge des Patienten P durch einen zu hohen Druck geschädigt wird, insbesondere bei einer manuellen Beatmung durch einen Atembeutel 26. Die Druckschranke, bei der dieses Überdruckventil 29 öffnet, lässt sich manuell von außen und / oder automatisch durch eine Ansteuerung des Überdruckventils 29 einstellen.

Ein Narkosemittelverdampfer 31 vermag einen Fluidstrom 28 mit einer dampfförmigen Mischung aus einem Trägergas und mindestens einem Narkosemittel in den Beatmungskreislauf 40 einzuspeisen. Das Trägergas umfasst bevorzugt Sauerstoff. Außerdem lässt sich dem Beatmungskreislauf 40 ein Fluidstrom 27 von Frischluft oder einem sonstigen frischen Gas zuführen.

Der Beatmungskreislauf 40 wird von der Pumpe 24a und optional von einem Atembeutel 26, der manuell betätigt werden kann, in Gang gehalten.

Die Pumpe 24a, der optionale Narkosemittelverdampfer 31, der Kohlendioxidabsorber 25a, die Rückschlagventile 23a und 23b sowie die Ventile 24b und 29 und der optionale Atembeutel 26 gehören zu einem nur schematisch gezeigten Beatmungsgerät 1, welches als Anästhesiegerät ausgestaltet sein kann. Möglich ist auch, dass der Beatmungskreislauf 40 ausschließlich mithilfe des Atembeutels 26 im Gang gehalten wird, beispielsweise an Bord eines Fahrzeugs oder einem anderen Ort, an dem keine stationäre Stromversorgung zur Verfügung steht oder an dem diese ausgefallen ist.

Ein nur schematisch gezeigtes signalverarbeitendes Beatmungs-Steuergerät (control unit) 35 empfängt Messwerte von einem Drucksensor 58, der den Luftdruck $P_{amb}$ in der Umgebung des Beatmungskreislaufs 40 misst, und ein Signal von einem optionalen Temperatursensor 39, der die Umgebungstemperatur misst. Außerdem empfängt das Beatmungs-Steuergerät 35 Messwerte von einem Drucksensor 36, der den aktuellen Druck im Beatmungskreislauf 40 misst, beispielsweise den am Patienten P anliegenden Beatmungsdruck (pressure in airway, $P_{aw}$), in einer Ausgestaltung als Differenzdruck relativ zum Umgebungsdruck $P_{amb}$. Das Beatmungs-Steuergerät 35 steuert die Pumpe 24a, den Narkosemittelverdampfer 31 und weitere Bestandteile des Beatmungskreislaufs 40 an, um eine gewünschte künstliche Beatmung und optional eine Narkotisierung des Patienten P zu realisieren.

Häufig wird gewünscht, dass das Gasgemisch Gg, welches dem Patienten P zugeführt wird, eine bestimmte Eigenschaft erfüllt. Insbesondere soll die Konzentration eines Bestandteils des Gasgemischs Gg in einem vorgegebenen Bereich liegen. Beispielsweise soll der Anteil von reinem Sauerstoff in einem vorgegebenen Bereich liegen, beispielsweise zwischen 40 Vol-% und 50 Vol-% oder auch zwischen 25 Vol-% und 30 Vol-%. Oder der Anteil von Narkosemittel soll in einem vorgegebenen Bereich liegen. Möglich ist, dass dieser vorgegebene Bereich zeitlich veränderlich ist. Außerdem soll häufig der tatsächliche Druck des Gasgemischs Gg einem vorgegebenen zeitlichen Druck-Verlauf folgen.

Insbesondere für die Ansteuerung des Beatmungsgeräts 1 ist es erforderlich, dass die tatsächliche aktuelle Konzentration von Kohlendioxid ($CO_2$) und / oder Sauerstoff ($O_2$) und / oder Lachgas ($N_2O$) und / oder des Drucks $P_{aw}$ und optional von eingespeistem Narkosemittel gemessen wird, und zwar die Konzentrationen und der Druck $P_{aw}$ an einer Messposition nahe der patientenseitigen Koppeleinheit 21 und somit nahe dem Mund und / oder der Nase des Patienten P.

Zu diesem Zweck wird eine Probe mit Atemgas (im Folgenden: eine Gasprobe Gp) über eine Gasprobe-Fluidführungseinheit (Sensor-Fluidführungseinheit) in Form eines Entnahmeschlauchs 52 aus dem Beatmungskreislauf 40 entnommen (abgezweigt), analysiert und über einen Abführschlauch 56 wieder in den Beatmungskreislauf 40 eingespeist. Der Entnahmeschlauch 52 beginnt in einem Abzweigpunkt 34 zwischen der patientenseitigen Koppeleinheit 21 und dem Y-Stück 22. Am Abzweigpunkt 34 befindet sich optional ein nicht gezeigtes Ventil, welches in geschlossener Stellung den Entnahmeschlauch 52 vom Beatmungskreislauf 40 abtrennt und welches sich vom Beatmungs-Steuergerät 35 ansteuern lässt. Bei vollständig geöffnetem oder fortgelassenem Ventil steht der Entnahmeschlauch 52 in einer nicht eingeschränkten Fluidverbindung mit dem Beatmungskreislauf 40. Der Abführschlauch 56 führt zu einem Einmündepunkt 37 flussaufwärts vom Kohlendioxidabsorber 25a.

Der Entnahmeschlauch 52 leitet die Gasprobe Gp zu einer Sensor-Anordnung 50. Diese Sensor-Anordnung 50 ist räumlich von der patientenseitigen Koppeleinheit 21 entfernt und gehört beispielsweise ebenfalls zu dem schematisch gezeigten Beatmungsgerät 1. In Figur 1 ist die Sensor-Anordnung 50 zur Verdeutlichung außerhalb des Beatmungsgeräts 1 gezeigt. Die Sensor-Anordnung 50 umfasst im Ausführungsbeispiel eine Pumpe 55, welche die Gasprobe Gp aus den Beatmungskreislauf 40 absaugt und durch den Entnahmeschlauch 52 ansaugt. Bevorzugt erzeugt die Pumpe 55 an der zum Entnahmeschlauch 52 hin zeigenden Seite dauerhaft einen Unterdruck und an der zum Abführschlauch 56 hin zeigenden Seite dauerhaft einen Überdruck. Die Pumpe 55 vermag einen Volumenfluss zu erzeugen, der bevorzugt zeitlich konstant ist und beispielsweise 200 ml/min beträgt.

Ein Sensor 54 vermag Signale zu erzeugen, welche mit der jeweiligen Konzentration von $CO_2$, $N_2O$ und Narkosemittel in der abgesaugten Gasprobe Gp korrelieren. Bevorzugt umfasst dieser Sensor 54 einen Infrarot-Messkopf, der das Dipol-Moment von Molekülen in der Gasprobe Gp ausnutzt und die Absorption von infrarot-aktiven Gasen quantitativ auswertet, um die jeweilige Konzentration zu bestimmen. Bevorzugt umfasst der Sensor 54 eine Strahlungsquelle, die elektromagnetische Strahlung emittiert, sowie einen Detektor, der ein Maß für die Intensität von auftreffender elektromagnetischer Strahlung misst und ein entsprechendes Signal erzeugt. Die Strahlung durchdringt die Gasprobe Gp, und ein zu detektierendes Gas absorbiert einen Teil der Strahlung.

Ein Sensor 53 vermag ein Signal zu erzeugen, welches u.a. mit der Konzentration von $O_2$ oder eines sonstigen parametrischen Bestandteils der Gasprobe Gp korreliert. Der Sensor 53 wird weiter unten näher beschrieben.

In einer Ausgestaltung befindet sich im Entnahmeschlauch 52 zeitweise Atemluft, die zum Patienten P gefördert wird, und zeitweise Atemluft, die der Patient P ausgeatmet hat. In einer Ausgestaltung wird die Faustformel verwendet, dass der Anteil von Sauerstoff in ausgeatmeter Luft um 5 Vol-% unter dem Anteil von Sauerstoff in der eingeatmeten Luft liegt.

Möglich ist, dass die Sensor-Anordnung 50 weitere Konzentrations-Sensoren umfasst, insbesondere um Redundanz bereitzustellen. Außerdem umfasst die Sensor-Anordnung 50 einen Drucksensor 57, der den Druck $P_{cell}$ der Gasprobe Gp am Eingang der Sensor-Anordnung 50 misst. Dieser Druck $P_{cell}$ ist zeitlich veränderlich, weil der Druck $P_{aw}$ im Beatmungskreislauf 40 variiert und weil der Entnahmeschlauch 52 mit dem Beatmungskreislauf 40 in einer Fluidverbindung steht, wenn am Abzweigpunkt 34 kein Ventil vorhanden ist oder solange das optionale Ventil am Abzweigpunkt 34 geöffnet ist. Bei fehlendem oder geöffneten Ventil pflanzt sich der Druck im Beatmungskreislauf 40 annähernd mit Schallgeschwindigkeit zu der Sensor-Anordnung 50 fort.

Die Sensor-Anordnung 50, eine nachfolgend beschriebene Signalverarbeitungseinheit 30, die Pumpe 55, eine nachfolgend beschriebene Wasserfalle 51 der Entnahmeschlauch 52 und der Abführschlauch 56 gehören zu einem Messsystem 100, das in Figur 1 schematisch angedeutet ist und zur Beatmungs-Anordnung 200 gehört.

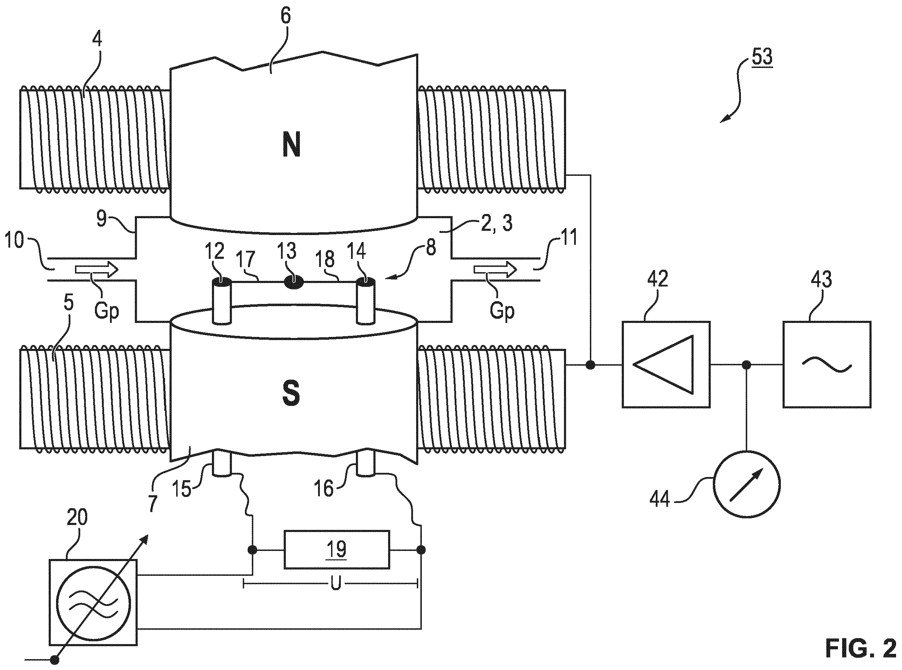

Nachfolgend wird mit Bezug auf Figur 2 eine erste Realisierungsform des Sauerstoff-Sensors 53 beschrieben. Die Ausgestaltung nutzt die Tatsache aus, dass Sauerstoff ein paramagnetisches Gas ist.

Zwei Polschuhe 6, 7 und zwei Feldspulen 4, 5 erzeugen ein Magnetfeld. Zwischen den beiden Polschulen 6, 7 tritt ein Luftspalt 3 auf, der als eine Messkammer 2 fungiert. Diese Messkammer 2 wird durch die beiden Polschuhe 6, 7 sowie einer Wandung 9 begrenzt. In die Wandung 9 sind ein Einlass 10 und ein Auslass 11 eingelassen. Die Gasprobe Gp fließt vom Einlass 10 durch die Messkammer 2 hindurch zum Auslass 11.

Ein Thermoelement 8 ist an zwei Verbindungsstellen 12 und 14 an zwei Stützdrähten 15, 16 befestigt, wobei die beiden Stützdrähte 15, 16 durch den unteren Polschuh 7 hindurchgeführt sind und in einem thermischen Kontakt mit dem unteren Polschuh 7 stehen. Das Thermoelement 8 umfasst zwei Drähte 17, 18, die an einer Verbindungsstelle 13 miteinander verbunden sind. Eine Spannungsquelle 20 legt eine Wechselspannung an die beiden Stützdrähte 15, 16 und damit an das Thermoelement 8 an. Durch den fließenden Strom wird das Thermoelement 8 auf eine Arbeitstemperatur erhitzt, die größer ist als die Temperatur der Gasprobe Gp in der Messkammer 2. An einem Messwiderstand 19 wird eine Spannung U gemessen. Diese Spannung U enthält einen Wechselstrom-Anteil und einen Gleichstrom-Anteil.

Die aktuelle Arbeitstemperatur des Thermoelements 8 wird an der Verbindungsstelle 13 gemessen. Diese Temperatur hängt einerseits von der Spannung U ab, die zwischen den Stützdrähten 15, 16 auftritt, und andererseits von der Wärmeleitfähigkeit der Gasprobe Gp in der Messkammer 2. Eine Regelung (closed-loop control) wird mit dem Ziel durchgeführt, dass die Arbeitstemperatur des Thermoelements 8 auf einen konstanten Wert geregelt wird. Die Stellgröße ist die zeitlich veränderliche Spannung U, welche die Spannungsquelle 20 an die Stützdrähte 15, 16 anlegt. Weil die Temperatur des Thermoelements 8 gleich bleibt, korreliert eine elektrische Detektionsgröße des Thermoelements 8 mit der Wärmeleitfähigkeit der Gasprobe Gp in der Messkammer 2. Möglich ist, dass die angelegte elektrische Spannung U selber die Detektionsgröße ist.

Eine andere Realisierungsform, um die Detektionsgröße zu messen, ist die folgende: Als Detektionsgröße wird die zeitlich veränderliche elektrische Leistung, die dem Thermoelement 8 zugeführt wird, gemessen. Die beiden Stützdrähte 15 und 16 sind durch den elektrischen Messwiderstand 19 elektrisch miteinander verbunden. Die elektrische Spannung U, die an diesem Messwiderstand 19 anfällt, wird gemessen. Außerdem wird ein Maß für die Stärke I des elektrischen Stroms, der durch das Thermoelement 8 fließt, gemessen. Die elektrische Leistung hängt bekanntlich von der Spannung U und der Stromstärke I ab.

Eine Spannungsquelle 43 ist über einen Leistungsverstärker 42 mit der Feldspule 5 verbunden, und die Feldspule 4 ist mit elektrischer Masse verbunden. Die Spannungsquelle 43 gibt eine oszillierende, insbesondere sinusförmige, elektrische Spannung ab. Diese Spannung erzeugt ein oszillierendes, insbesondere sinusförmig variierendes Magnetfeld in der Messkammer 2. Das Thermoelement 8, das auf eine gleichbleibende Temperatur erhitzt wird, gibt an die Gasprobe Gp in der Messkammer 2 eine Wärmemenge pro Zeiteinheit ab. Diese abgegebene Wärmemenge pro Zeiteinheit korreliert mit der gemessenen elektrischen Leistung, die dem Thermoelement 8 zugeführt wird. Weil die Feldstärke des Magnetfelds in der Messkammer 2 periodisch oszilliert, oszilliert auch die zugeführte Wärmemenge pro Zeiteinheit - vorausgesetzt ein paramagnetisches Gas befindet sich als Teil der Gasprobe Gp in der Messkammer 2. Der zeitliche Verlauf der Wärmeleitfähigkeit enthält eine magnetisch modulierte Wärmeleitfähigkeit, das ist ein Anteil, der mit der Stärke des angelegten Magnetfelds oszilliert. Durch eine entsprechende Filterung lassen sich zwei Signale herleiten, nämlich ein Maß für die magnetisch modulierte Wärmeleitfähigkeit und ein Maß für die gesamte Wärmeleitfähigkeit der Gasprobe Gp.

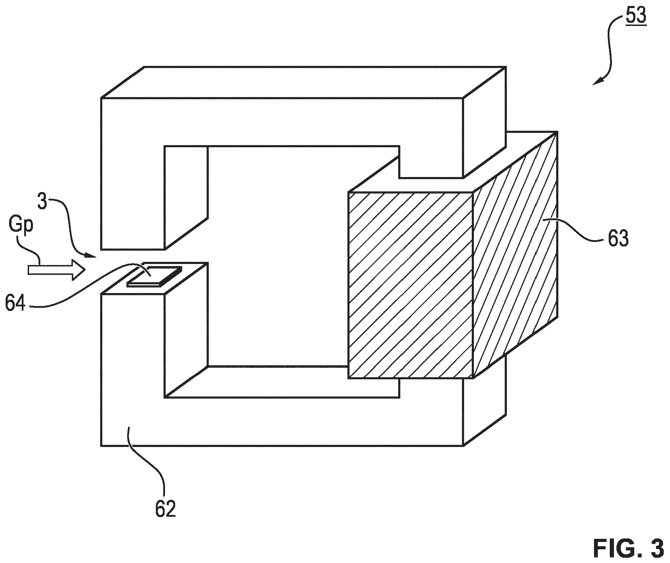

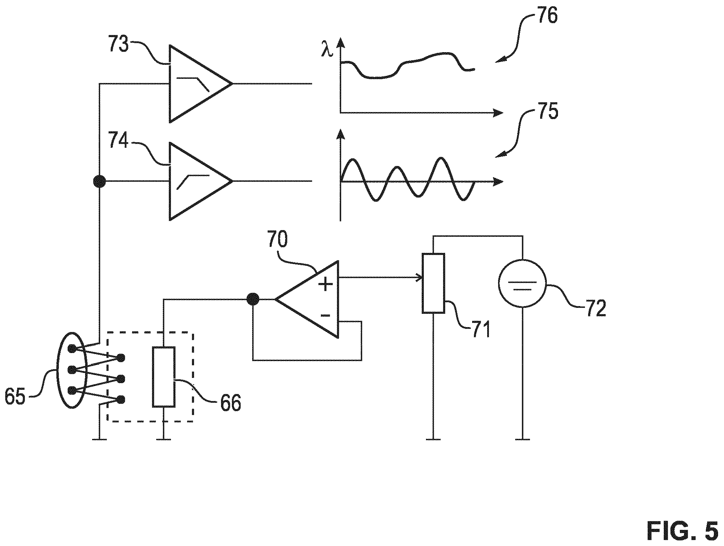

Figur 3, Figur 4 und Figur 5 zeigen eine weitere Ausgestaltung des Sensors 53. Gleiche Bezugszeichen haben die gleichen Bedeutungen wie in Figur 2. Die Schaltung von Figur 5 lässt sich entsprechend auch für die Ausgestaltung gemäß Figur 2 anwenden.

Ein Elektromagnet 62 mit einer elektrischen Spule 63 erzeugt in dem Luftspalt 3 ein zeitlich veränderliches Magnetfeld, vgl. Figur 3. Eine zu untersuchende Gasprobe Gp erreicht diesen Luftspalt 3. Der zeitliche Verlauf der Stärke des erzeugten Magnetfeldes wird durch die Ansteuerung der Spule 63 festgelegt. Bevorzugt hat die Stärke des Magnetfelds einen oszillierenden, insbesondere sinusförmigen Verlauf. Im Luftspalt 3 ist eine Messeinheit 64 angeordnet, die als Chip implementiert ist, bevorzugt als Halbleiter-Chip, der beispielsweise unter Verwendung von dotiertem Silizium hergestellt ist.

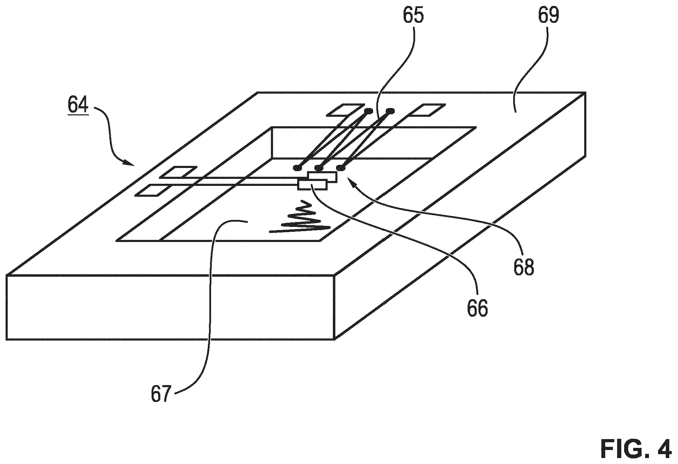

Figur 4 zeigt die Messeinheit 64 im Detail. Die Messeinheit 64 umfasst ein elektrisch steuerbares Wärmeleitungs-Messelement 65, ein elektrisch steuerbares Heizelement 66 und eine Membrane 67 in einem Rahmen 69. Eine Gasprobe Gp kann durch Löcher in der Membrane 67 oder um die Membrane 67 herum zu den Elementen 65, 66 gelangen. Das Heizelement 66 kann eine elektrisch leitfähige Widerstandsstruktur sein, die auf der Membrane 67 abgeschieden ist, oder als Heizdraht ausgestaltet sein. Die beiden Elemente 65 und 66 können auch als ein einstückiges Element ausgestaltet sein, welches einen temperaturabhängigen elektrischen Widerstand aufweist.

Das Heizelement 66 heizt das Messelement 65 auf eine Arbeitstemperatur auf, die größer als die Temperatur einer Gasprobe Gp in der Messkammer 2 ist. Das beheizte Messelement 65 misst die Temperatur an der Messstelle 68. In der gezeigten Realisierungsform misst das Messelement 65 eine Thermospannung und nutzt den Seebeck-Effekt aus. Die Wärmeleitfähigkeit der Gasprobe Gp an der Messstelle 68 verändert sich synchron zu dem zeitlich variierenden Magnetfeld, welches der Elektromagnet 62 erzeugt, vorausgesetzt, die Gasprobe Gp an der Messstelle 68 enthält mit ausreichend großer Konzentration ein paramagnetisches Gas. Eine höhere Wärmeleitfähigkeit führt dazu, dass die Wärmeenergie besser weggeleitet wird. Dies wiederum bewirkt eine niedrigere Temperatur, welche wiederum eine niedrigere Thermospannung bewirkt.

Figur 5 zeigt beispielhaft eine Auswerteschaltung mit folgenden Bestandteilen:

- einem Verstärker 70, der als Impedanzwandler geschaltet ist,
- einem Spannungsteiler 71 mit variablem Abgriff,
- eine Gleichspannungsquelle 72,
- einem Tiefpass-Filter 73 und
- einen Hochpass-Filter 74, der parallel zum Tiefpass-Filter 73 geschaltet ist.

Das Heizelement 66 ist über den Verstärker 70 und den Spannungsteiler 71 mit der Gleichspannungsquelle 72 verbunden. Das Ausgangssignal des Wärmeleitungs-Messelements 65 wird über den Tiefpass-Filter 73 sowie über den Hochpass-Filter 74 geführt. Am Ausgang des Hochpass-Filters 74 liegt ein oszillierendes, insbesondere sinusförmiges, also periodisch schwankendes Signal 75 an. Die Oszillation des Signals 75 wird durch die Oszillation des angelegten Magnetfelds bestimmt. Das Signal 75 korreliert mit der magnetisch modulierten Wärmeleitfähigkeit und damit mit dem Anteil des paramagnetische Gases, beispielsweise mit dem Anteil von Sauerstoff, in der Gasprobe Gp. Am Ausgang des Tiefpass-Filters 73 liegt ein Signal 76 an, welches nicht oder zumindest nicht abhängig von der Oszillation des Magnetfelds periodisch schwankt und mit der gesamten Wärmeleitfähigkeit der Gasprobe Gp korreliert.

Die Wärmemenge pro Zeiteinheit umfasst eine Überlagerung eines zeitlich gleichbleibenden Anteils und eines periodischen schwankenden Anteils. Der periodisch schwankende Anteil korreliert mit der Wärmeleitfähigkeit und damit mit der Konzentration von Sauerstoff in der Messkammer 2. Der gleichbleibende Anteil, also der Anteil, der nicht mit der Stärke des Magnetfelds oszilliert, korreliert mit der Wärmeleitfähigkeit der gesamten Gasprobe Gp in der Messkammer 2. Zu dieser Wärmeleitfähigkeit der Gasprobe Gp tragen die Wärmeleitfähigkeit der Sauerstoffs und die jeweilige Wärmeleitfähigkeit jedes anderen Bestandteils der Gasprobe Gp bei. Beide Anteile werden gemessen. Der Sensor 53 liefert also zwei Signale:

- ein periodisch schwankendes Signal, welches ein Maß für die Konzentration von Sauerstoff oder einem sonstigen paramagnetischen Gas in der Messkammer 2 ist (periodisch schwankender Anteil, Wechselspannung, resultiert aus der magnetisch modulierten Wärmeleitfähigkeit) und
- ein Signal für die gesamte Wärmeleitfähigkeit der Gasprobe Gp in der Messkammer 2 (gleichbleibender Anteil, Gleichspannung).

Optional werden ein gemessener Wert für die Konzentration eines Bestandteils und / oder für die Wärmeleitfähigkeit auf einer Anzeigevorrichtung 44 angezeigt, vgl. Figur 2.

Der Drucksensor 57 der Sensor-Anordnung 50 misst den gesamten absoluten Druck $P_{cell,abs}$ der Gasprobe Gp. Der Quotient aus einem Partialdruck und dem gesamten absoluten Druck $P_{cell,abs}$ liefert ein Maß für die Konzentration eines Bestandteils der abgesaugten Gasprobe Gp. Außerdem liefert der Drucksensor 57 ein Maß für den Druck der Gasprobe Gp.

Bevorzugt misst der Drucksensor 57 der Sensor-Anordnung 50 einen absoluten Druck $P_{cell,abs}$. Im Folgenden wird als Druck $P_{cell}$ der interne Druck in der Sensor-Anordnung 50 relativ zu dem Umgebungsdruck $P_{amb}$ verwendet, also $P_{cell} = P_{cell,abs} - P_{amb}$. Der relative Druck $P_{cell}$ kann daher auch negative Werte annehmen, nämlich bei einem Unterdruck in der Sensor-Anordnung 50 relativ zum Umgebungsdruck $P_{amb}$. Bevorzugt wird der relative Druck mehrmals während eines Atmungsvorgangs gemessen, und als Druck $P_{cell}$ wird ein Wert verwendet, der durch geeignete Mittelung über diese während eines Atmungsvorgangs gewonnenen Messwerte berechnet wurde.

Eine in Figur 1 schematisch gezeigte Signalverarbeitungseinheit 30 (control unit) empfängt Messwerte von Sensoren der Sensor-Anordnung 50, insbesondere von dem $CO_2$-, $N_2O$- und Narkosemittel-Sensor 54, von dem Wärmeleitungs- und $O_2$-Sensor 53, von den Drucksensoren 57 und 58 sowie von weiteren Sensoren, insbesondere vom Temperatursensor 39, und wertet diese Messwerte automatisch aus. Die Signalverarbeitungseinheit 30 erzeugt abhängig von den empfangenen und verarbeiteten Messwerten Signale betreffend die jeweilige aktuelle Konzentration von $O_2$, $CO_2$, $N_2O$ und / oder Narkosemittel sowie betreffend den Druck $P_{cell}$ und übermittelt diese Signale an das Beatmungs-Steuergerät 35. Das Beatmungs-Steuergerät 35 verwendet diese empfangenen Signale, um den Beatmungskreislauf 40 automatisch zu steuern (open-loop control) oder zu regeln (closed-loop control).

Die Gasprobe Gp, welche bevorzugt kontinuierlich von der Pumpe 55 angesogen wird, fließt durch eine schematisch gezeigte Wasserfalle 51 hindurch, welche flussaufwärts von den Sensoren 53 und 54 angeordnet ist. Diese Wasserfalle 51 ist mit mindestens einer gasdurchlässigen Membrane ausgestaltet, wobei diese Membrane bevorzugt aus einem chemisch trägen Werkstoff, z.B. Polytetrafluorethylen (PTFE), gefertigt ist. Diese Wasserfalle 51 kann beispielsweise so wie in DE 10 2007 046 533 B3 oder in DE 10 2009 024 040 A1 beschrieben aufgebaut sein. Auf diese Weise wird die angesaugte Gasprobe Gp von Kondensat, Partikeln, Schwebstoffen und Keimen befreit. Flüssigkeit, insbesondere auskondensierter Wasserdampf, wird von der Membrane zurückgehalten und fließt in einen Tank der Wasserfalle 51.

Möglich ist, dass auf dem Weg von dem Abzweigpunkt 34 des Entnahmeschlauchs 52 bis zu den Sensoren 54 und 53 ein Leck auftritt, beispielsweise weil der Entnahmeschlauch 52 nicht richtig mit der patientenseitigen Koppeleinheit 21, dem Y-Stück 22 oder der Wasserfalle 51 verbunden ist oder weil eine Materialermüdung oder eine Berührung oder sonstige mechanische Einwirkung von außen zu einem Leck geführt hat. Dieses Leck kann schlagartig auftreten, beispielsweise aufgrund eines mechanischen Einflusses oder weil die künstliche Beatmung begonnen wird, obwohl zwei Teile fälschlicherweise nicht fluiddicht miteinander verbunden sind, oder allmählich, beispielsweise aufgrund von Materialermüdung. In Figur 1 wird beispielhaft ein Leck L im Übergang zwischen dem Entnahmeschlauch 52 und der Wasserfalle 51 gezeigt.

Ein solches Leck L kann die Messergebnisse der Sensoren 53 und 54 und von optionalen weiteren Sensoren verfälschen. Denn im Entnahmeschlauch 52 tritt wenigstens bei der Exspiration ein Unterdruck relativ zum Umgebungsdruck $P_{amb}$ und auch relativ zum Druck $P_{aw}$ im Beatmungskreislauf 40 auf. Dieser Unterdruck resultiert daher, dass die Pumpe 55 des Messsystems 100 kontinuierlich eine Gasprobe Gp absaugt, und beträgt beispielsweise 100 hPa. Durch dieses Leck L hindurch kann wegen des Unterdrucks relativ zur Umgebung Umgebungsluft in den Entnahmeschlauch 52 oder in die Sensor-Anordnung 50 hinein gesaugt werden. Der Unterdruck hängt vom aktuellen und zeitlich variierenden Druck im Atmungskreislauf 40 ab. Weil der Unterdruck im Entnahmeschlauch 52 relativ zum Umgebungsdruck $P_{amb}$ variiert, variiert bei einem Leck L in der Regel auch die Menge der eingesaugten Umgebungsluft über der Zeit.

Die eingesogene Umgebungsluft kann beispielsweise eine höhere oder auch eine niedrigere Sauerstoffkonzentration im Beatmungskreislauf 40 als die tatsächliche Sauerstoffkonzentration vortäuschen und daher zu einer falschen Messung führen. Diese falsche Messung könnte zu einem Fehler bei der künstlichen Beatmung des Patienten P führen. Daher muss ein Leck L so schnell wie möglich erkannt werden, und ein entsprechender Alarm muss ausgegeben werden, um das Leck L rasch lokalisieren und beseitigen zu können. Andererseits wird gewünscht, möglichst wenige Fehlalarme zu generieren, idealerweise keine Fehlalarme.

Nachfolgend wird beispielhaft beschrieben, wie ein solches Leck L detektiert wird. Mit dem Begriff "Volumenfluss" wird das Volumen pro Zeiteinheit bezeichnet, welches durch eine Fluidführungseinheit fließt. Folgende Symbole werden verwendet:

| | |
|---|---|
| Vol'(50) | bekannter oder gemessener Volumenfluss, den die Pumpe 55 erzeugt und mit dem ein Gasgemisch Gg zur Sensor-Anordnung 50 fließt, ist bevorzugt zeitlich konstant und ist gleich der Summe Vol'(40) + Vol'(env) |
| Vol'(40) | Volumenfluss, der aus dem Beatmungskreislauf 40 abgesaugt wird und durch den Entnahmeschlauch 52 fließt, ist bei einem leckfreien Zustand gleich Vol'(50) |
| Vol'(env) | Volumenfluss, der bei Auftreten eines Lecks L aus der Umgebung in den Entnahmeschlauch 52 oder in die Sensor-Anordnung 50 gesaugt wird, ist bei einem leckfreien Zustand gleich Null |
| con(50) | gemessene Konzentration von Sauerstoff in der Gasprobe Gp, die die Sensor-Anordnung 50 erreicht, ist ein gewichtetes Mittel der Konzentrationen con(40) und con(env) und ist bei einem leckfreien Zustand gleich con(40) |
| con(40) | Konzentration von Sauerstoff im Beatmungskreislauf 40 am Abzweigpunkt 34, ist bei einem leckfreien Zustand gleich con(50) |
| con(env) | bekannte Konzentration von Sauerstoff in der Umgebungsluft, beträgt beispielsweise 20,95 Vol-% |
| con(rel) | Maß für den relativen Leck-Volumenfluss Vol'(rel), das abhängig von der Sauerstoff-Konzentration con(50) der Gasprobe Gp ermittelt wird, ist bei einem leckfreien Zustand gleich Null |
| Vol'(rel) | relativer Leck-Volumenfluss, nachfolgend erläutert |

Weil die Pumpe 55 der Sensor-Anordnung 50 die Gasprobe Gp ansaugt, bewirkt das Leck L, dass zusätzlich zur Gasprobe Gp aus dem Beatmungskreislauf 40 Luft aus der Umgebung angesaugt wird und die Sensor-Anordnung 50 erreicht. Daher erreicht eine Überlagerung von zwei Volumenflüssen die Sensor-Anordnung 50, nämlich

- ein Volumenfluss Vol'(40) aus dem Beatmungskreislauf 40 durch den Entnahmeschlauch 52 hindurch (gewünscht) und
- ein Volumenfluss Vol'(env) aus der Umgebung durch das Leck L hindurch (unerwünscht).

Also gilt

$$(1) \qquad Vol'(50) = Vol'(40) + Vol'(env)$$

Mit den Begriff "relativer Leck-Volumenfluss" Vol'(rel) wird der Anteil des Volumenflusses Vol'(env) durch das Leck L hindurch an dem gesamten Volumenfluss Vol'(50) zur Sensor-Anordnung 50 bezeichnet, also

$$(2) \qquad \text{Vol'(rel)} = \text{Vol'(env)} / \text{Vol'(50)}.$$

Falls kein Leck L aufgetreten ist, so gilt Vol'(50) = Vol'(40) und Vol'(rel) = 0. Ein rasch auftretendes Leck L führt zu einem raschen Anstieg des relativen Leck-Volumenflusses Vol'(rel).

Nachfolgend werden zwei verschiedene Maße für den relativen Leck-Volumenfluss Vol'(rel) beschrieben. Beide Maße werden laufend gemessen, um zu prüfen, ob ein Leck aufgetreten ist. Beide Maße beruhen jeweils auf einer Messgröße. Idealerweise stimmen die beiden Maße überein, in der Praxis differieren sie voneinander. Die Ausgestaltung, dass mindestens zwei Maße gemessen werden, erhöht die Zuverlässigkeit, dass jedes Leck L detektiert wird und kein Fehlalarm erzeugt wird. Die Ausgestaltung, dass mindestens zwei Maße für den relativen Leck-Volumenfluss Vol'(rel) verwendet werden, hat außerdem folgenden Vorteil: Beim Auftreten eines Lecks steigt der relative Leck-Volumenfluss Vol'(rel) an, egal, ob die Messgröße selber durch das Leck L ansteigt oder abfällt.

Im Ausführungsbeispiel beruht das eine Maß auf der Sauerstoff-Konzentration und wird mit con(rel) bezeichnet. Allgemein ist der Volumenfluss eines Bestandteils eines Gasgemischs durch eine Fluidführungseinheit hindurch das Produkt aus dem gesamten Volumenfluss durch die Fluidführungseinheit hindurch und dem als Vol-% gemessenen Anteil des Bestandteils am Gasgemisch. Der Volumenfluss von Sauerstoff in die Sensor-Anordnung 50 resultiert aus einem Volumenfluss von Sauerstoff aus dem Beatmungskreislauf 40 und dann, wenn ein Leck L aufgetreten ist, einem Volumenfluss von Sauerstoff aus der Umgebung durch das Leck L hindurch. Daher gilt für die zugeführte Sauerstoff-menge pro Zeiteinheit:

$$(3) \qquad \text{Vol'(50)*con(50)} = \text{Vol'(40)*con(40)} + \text{Vol'(env)*con(env)} =$$
$$[1\text{-Vol'(rel)}]\text{*Vol'(50)*con(40)} + \text{Vol'(rel)\_Vol'*con(env)}.$$

Durch eine Umformung folgt, dass für den relativen Leck-Volumenfluss Vol'(rel) gilt:

$$(4) \qquad \text{Vol'(rel)} = \text{Vol'(env)} / \text{Vol'(40)} = [\text{con(40)} - \text{con(50)}] / [\text{con(40)} - \text{con(env)}].$$

Diese Gleichung gilt nur idealerweise. Die rechte Seite wird als erstes Maß für den relativen Leck-Volumenfluss Vol'(rel) verwendet, also

$$(5) \qquad \text{con(rel)} = [\text{con(40)} - \text{con(50)}] / [\text{con(40)} - \text{con(env)}].$$

Die Sauerstoffkonzentration con(env) in Luft ist bekannt und wird vorgegeben. Der Sensor 53 misst laufend die Konzentration con(50) von Sauerstoff in der Sensor-Anordnung 50.

In einer Ausgestaltung misst ein nicht gezeigter Sauerstoffsensor die tatsächliche Sauerstoff-Konzentration con(40) im Beatmungskreislauf 40. Häufig wird eine gewünschte Sauerstoff-Konzentration im Beatmungskreislauf 40 vorgegeben. Abhängig von der vorgegebenen gewünschten Sauerstoff-Konzentration wird Sauerstoff in den Beatmungskreislauf 40 eingespeist. Optional misst ein nicht gezeigter Sauerstoffsensor im Beatmungsgerät 1 die tatsächliche Sauerstoff-Konzentration con(40) im eingespeisten Gasgemisch Gg. Die vorgegebene oder gemessene Sauerstoff-Konzentration wird als die Sauerstoff-Konzentration con(40) verwendet. Möglich ist auch, dass davon ausgegangen wird, dass zu Beginn der künstlichen Beatmung kein Leck L aufgetreten ist. Dann sind con(50) = con(40) und Vol'(rel) = 0. Falls plötzlich ein Leck L auftritt, so steigt der relative Leck-Volumenfluss Vol'(rel) rasch an.

Gemäß der gerade beschriebenen Realisierungsform misst der oben mit Bezug auf Figur 2 bis Figur 5 beschriebene Sensor 53 laufend ein Maß für die Konzentration con(50) von Sauerstoff in der abgesaugten Gasprobe Gp, welche die Sensor-Anordnung 50 erreicht. Der Sensor 53 kann auch ein anderes geeignetes Messprinzip anwenden. Anstelle der Konzentration von Sauerstoff kann auch die Konzentration eines anderen Bestandteils dieser Gasprobe Gp laufend gemessen werden.

Die Erkennung eines Lecks wird erfindungsgemäß nicht oder wenigstens nicht nur auf die Veränderung der Sauerstoff-Konzentration gestützt. Denn eine rasche Veränderung des relativen Leck-Volumenflusses Vol'(rel) kann auch eine andere Ursache als ein Leck haben, beispielsweise eine gewollte Veränderung während der künstlichen Beatmung, insbesondere eine Okklusion, bei welcher die Versorgung des Patienten P mit Atemluft sehr kurz unterbrochen wird, um einen Vitalparameter des Patienten P zu messen. Daher wird im Ausführungsbeispiel zusätzlich eine weitere zeitlich veränderliche Eigenschaft der Gasprobe Gp überwacht. Erfindungsgemäß ist diese weitere Eigenschaft die relative Wärmeleitfähigkeit, und eine rasche zeitliche Veränderung der ermittelten relativen Wärmeleitfähigkeit der Gasprobe Gp wird detektiert. Möglich ist auch, die Überwachung, ob ein Leck aufgetreten ist, ausschließlich auf die Wärmeleitfähigkeit zu stützen.

Nachfolgend werden folgende Bezeichnungen verwendet:

| WLF(40) | Wärmeleitfähigkeit der Gasprobe Gp, die aus dem Beatmungskreislauf 40 durch den Entnahmeschlauch 52 hindurch abgesaugt wird |
|---|---|
| WLF(env) | bekannte oder gemessene Wärmeleitfähigkeit der Umgebungsluft |
| WLF(50) | Wärmeleitfähigkeit der Gasprobe Gp, welche die Sensor-Anordnung 50 erreicht, wird vom Sensor 53 gemessen |
| WLF(rel) | Maß für den relativen Leck-Volumenfluss Vol'(rel), das abhängig von der Wärmeleitfähigkeit WLF(50) der Gasprobe Gp ermittelt wird |

Falls kein Leck L aufgetreten ist, so gilt WLF(50) = WLF(40) und WLF(rel) = 0.

In einer Ausgestaltung wird die Annahme getroffen, dass die Wärmeleitfähigkeit WLF(50) der Gasprobe Gp, welches die Sensor-Anordnung 50 erreicht, ein gewichtetes Mittel der Wärmeleitfähigkeit WLF(40) der Gasprobe Gp, die aus dem Beatmungskreislauf 40 abgesaugt ist, und der Wärmeleitfähigkeit WLF(env) der Umgebungsluft ist. Der Gewichtsfaktor für die Wärmeleitfähigkeit WLF(env) der Umgebungsluft ist der relative Leck-Volumenfluss Vol'(rel), der Gewichtsfaktor für die Wärmeleitfähigkeit des Gasgemischs Gg im Beatmungskreislauf 40 daher 1- Vol'(rel).

$$(6) \qquad WLF(50) = Vol'(rel) * WLF(env) + [1- Vol'(rel)] * WLF(40).$$

Das Maß WLF(rel) für den relativen Leck-Volumenfluss Vol'(rel), das auf der Wärmeleitfähigkeit beruht, ist idealerweise gleich Vol'(rel) und wird gemäß der nachfolgenden Rechenvorschrift berechnet, die sich ergibt, wenn man die Formel (6) nach Vol'(rel) auflöst:

$$(7) \qquad WLF(rel) = [WLF(40) - WLF(50)] / [WLF(40) - WLF(env)].$$

Die Wärmeleitfähigkeit WLF(env) von trockener Umgebungsluft ist bekannt, sie beträgt bei 1 bar und 15 Grad C $\lambda$ = 0,02603 W / mK. Der mit Bezug auf Figur 2 bis Figur 5 beschriebene Sensor 53 misst die Wärmeleitfähigkeit WLF(50) der Gasprobe Gp, welches die Sensor-Anordnung 50 erreicht. In einer Ausgestaltung wird der Gleichstrom-Anteil (der gleichbleibende Anteil) der Spannung U, die am Thermoelement 8 von Figur 2 anliegt, oder der Gleichstrom-Anteil (Signal 76) von Figur 5 als Maß für die Wärmeleitfähigkeit WLF(50) verwendet. Die Wärmeleitfähigkeit WLF(40) im Beatmungskreislauf 40 unterscheidet sich in der Regel deutlich von der Wärmeleitfähigkeit WLF(env) in der Umgebung, so dass der Nenner nicht Null ist. Die Wärmeleitfähigkeit WLF(40) wird beispielsweise von einem Sensor im Beatmungsgerät 1 gemessen. In vielen Fällen ist auch die Annahme gerechtfertigt, dass zu Beginn der künstlichen Beatmung kein Leck vorhanden ist und daher anfangs WLF(40) = WLF(50) gilt.

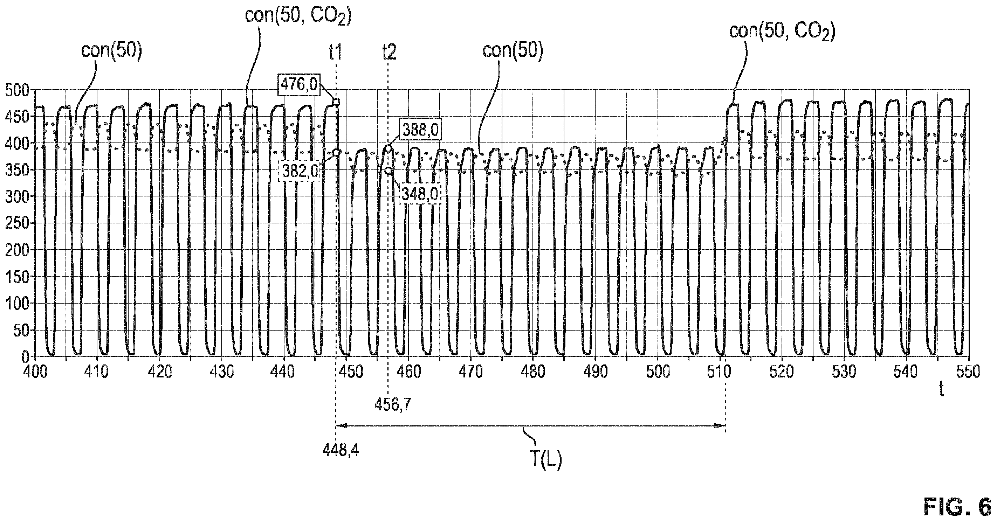

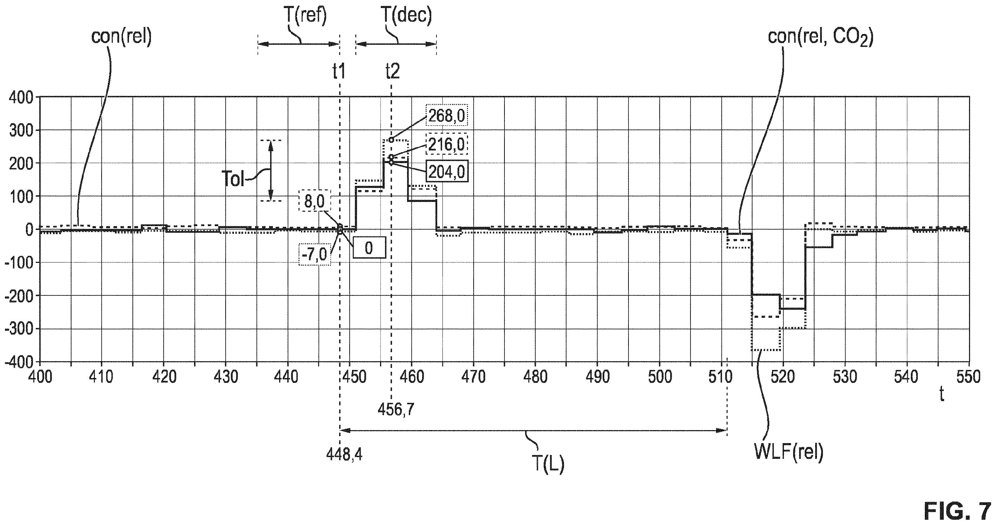

Figur 6 und Figur 7 zeigen beispielhaft Versuchsergebnisse, welche die Erfinder in internen Versuchen erzielt haben. Auf der x-Achse ist jeweils die Zeit t eingetragen. In diesen internen Versuchen wurden die Konzentrationen von $O_2$ und $CO_2$ sowie die Wärmeleitfähigkeit WLF gemessen und verwendet.

Auf der y-Achse von Figur 6 sind die zeitlichen Verläufe der gemessenen Sauerstoff-Konzentration con(50) und der gemessenen Kohlendioxid-Konzentration con(50, $CO_2$) in der abgezweigten Gasprobe Gp in 1/10 Vol-% aufgetragen. Der Sensor 53 von Figur 2 bis Figur 5 oder auch ein anderer geeigneter Sensor misst wiederholt die Sauerstoff-Konzentration con(50), der Sensor 54 von Figur 1 die Kohlendioxid-Konzentration con(50, $CO_2$). Wie zu sehen ist, sind sowohl die gemessene Sauerstoff-Konzentration con(50) als auch die gemessene Kohlendioxid-Konzentration con(50, $CO_2$) im Zeitraum T(L) geringer als im Rest des Messzeitraums. Diese Beobachtung ist ein Indiz für ein Leck L, kann aber auch eine andere Ursache haben. Beispielhaft sind die beiden jeweiligen Messwerte für die Zeitpunkte t1 = 448,4 und t2 = 456,7 eingetragen.

In der Regel bewirkt ein Leck L, dass die Wärmeleitfähigkeit der Gasprobe Gp, die die Sensor-Anordnung 50 erreicht, steigt. Ein Grund ist der, dass Umgebungsluft in der Regel eine höhere Wärmeleitfähigkeit als diejenigen Bestandteile, die außer Sauerstoff im Beatmungskreislauf 40 enthalten sind, aufweist. In einzelnen Fällen kann das Leck aber auch zu einer kleineren Wärmeleitfähigkeit führen. Die Erfindung erspart die Notwendigkeit, eine entsprechende Fallunterscheidung und die erforderliche Messung vornehmen zu müssen.

In dem Beispiel von Figur 7 werden die zeitlichen Verläufe von folgenden drei Maßen für den relativen Leck-Volumenfluss Vol'(rel) gezeigt:

- das Maß con(rel), das gemäß der Rechenvorschrift (5) berechnet wird,

- das Maß WLF(rel), das gemäß der Rechenvorschrift (7) berechnet wird, und
- ein weiteres Maß con(rel, $CO_2$) für den relativen Leck-Volumenfluss Vol'(rel), das abhängig von der Kohlendioxid-Konzentration berechnet wird, wofür eine Rechenvorschrift analog zur Rechenvorschrift (5) angewendet wird.

Wie in Figur 7 zu sehen ist, bleiben alle drei Maße zeitlich konstant, solange kein Leck aufgetreten ist. Denn das Beatmungsgerät 1 verändert im gezeigten Zeitraum nicht die Konzentration eines Bestandteils in dem Gasgemisch Gg, welches durch den Beatmungskreislauf 40 fließt. Daher ändert sich auch die Wärmeleitfähigkeit des Gasgemischs Gg und damit der abgezweigten Gasprobe Gp nicht. Zu Beginn des Zeitraums T(L), in dem das Leck L auftritt, verändern sich alle drei Maße, und zwar in die gleiche Richtung und etwa um den gleichen Betrag. Zu Beginn des nachfolgenden Zeitraums verändern sich alle drei Maße ebenfalls in die gleiche Richtung.

Das Beispiel von Figur 7 zeigt drei Maße für den relativen Leck-Volumenfluss Vol'(rel). Erfindungsgemäß wird mindestens ein Maß für den relativen Leck-Volumenfluss Vol'(rel) verwendet, bevorzugt werden mindestens zwei Maße verwendet, nämlich ein Maß WLF(rel), das auf der Wärmeleitfähigkeit beruht, und mindestens ein Maß, das auf der Konzentration eines Bestandteils der Gasprobe Gp oder dem Druck $P_{cell}$ (nicht gezeigt) der Gasprobe Gp in der Sensor-Anordnung 50 beruht.

Idealerweise gilt, dass das oder jedes Maß für den relativen Volumenfluss Vol'(rel) gleich dem tatsächlichen relativen Leck-Volumenfluss ist. Idealerweise gilt also zu jedem Zeitpunkt t

$$con(rel)(t) = con(rel, CO_2)(t) = WLF(rel)(t) = Vol'(rel)(t).$$

In der Regel differiert aber das oder jedes Maß von dem tatsächlichen relativen Leck-Volumenfluss Vol'(rel), insbesondere aufgrund von Messfehlern und weil sich die jeweilige Messgröße nur mit einer begrenzten Geschwindigkeit fortpflanzt. Daher wird im Ausführungsbeispiel das nachfolgend beschriebene Überwachungs-Verfahren angewendet, um zu mit größerer Sicherheit entscheiden, ob tatsächlich ein Leck L aufgetreten ist oder nicht.

Vorgegeben wird eine Mindest-Zeitdauer ΔT. Geprüft wird, ob in einem Entscheidungs-Zeitraum T(dec), welcher mindestens so lang wie die Mindest-Zeitdauer ΔT ist, die verwendeten Maße con(rel), con(rel, $CO_2$), WLF(rel) für den relativen Leck-Volumenfluss Vol'(rel) folgende Kriterien kumulativ erfüllen:

- Im gesamten Entscheidungs-Zeitraum T(dec) vergrößert sich jedes Maß con(rel), con(rel, $CO_2$), WLF(rel) relativ zu einen Referenz-Zeitraum T(ref) vor dem Entscheidungs-Zeitraum, wobei die absolute oder relative Veränderung größer als einer vorgegebenen unteren Schranke ist.
- Im gesamten Entscheidungs-Zeitraum T(dec) weichen die Maße um nicht mehr als eine vorgegebene Schranke absolut oder relativ voneinander ab.

In Figur 7 werden beispielhaft der Entscheidungs-Zeitraum T(dec) und der vorhergehende Referenz-Zeitraum T(ref) gezeigt. Der Entscheidungs-Zeitraum T(dec) ist kürzer als der Zeitraum T(L), in dem das Leck L aufgetreten ist. Außerdem wird in Figur 7 ein Toleranzband Tol gezeigt. Im Entscheidungs-Zeitraum T(dec) liegen alle drei Maße in diesem Toleranzband Tol. Die Breite dieses Toleranzbands Tol ist vorgegeben.

Gemäß der Erfindung wird mindestens ein Maß für den relativen Leck-Volumenfluss Vol'(rel) ermittelt und analysiert. Bevorzugt werden mehrere Maße miteinander verglichen. Durch dieses Vorgehen wird ein Indiz für ein Leck detektiert. Dieses Indiz stammt mit einer relativ hohen Zuverlässigkeit tatsächlich von einem Leck. Möglich sind aber Fehlalarme, insbesondere weil ein anderes Ereignis als ein Leck L den relativen Leck-Volumenfluss Vol'(rel) signifikant verändert. Möglich ist also, dass zwar ein Indiz für ein Leck detektiert wird, aber in Wirklichkeit kein Leck aufgetreten ist.

In einer Ausgestaltung wird dann, wenn so wie gerade beschrieben ein Indiz für ein Leck detektiert ist, ein Alarm ausgegeben. Ein Benutzer kann dann die Fluidverbindungen überprüfen.

Bevorzugt wird hingegen zunächst automatisch eine Überprüfungs-Abfolge durchgeführt und ausgelöst. Wenn diese Überprüfungs-Abfolge erbringt, dass tatsächlich ein Leck L aufgetreten ist, so wird ein entsprechender Alarm ausgegeben. Diese Überprüfungs-Abfolge führt aber dazu, dass die Sensor-Anordnung 50 zeitweise keine Signale zu liefern vermag. Daher wird die Überprüfungs-Abfolge bevorzugt nur dann durchgeführt, wenn ein Indiz für ein Leck L detektiert ist.

Die Überprüfungs-Abfolge umfasst die folgenden Schritte:

- Für die Dauer der Überprüfung wird die Pumpe 55 abgeschaltet.
- Ein optionales Ventil zwischen dem Entnahmeschlauch 52 und den Beatmungskreislauf 40 nahe dem Abzweigpunkt 34 wird oder bleibt geöffnet.
- Bevorzugt wird ein nicht gezeigtes optionales Ventil zwischen den Sensoren 53 und 54 geschlossen.
- Falls kein Leck vorhanden ist, so ist der Druck in dem Segment zwischen dem Abzweigpunkt 34 und dem Sensor 54 gleich den Druck im Beatmungskreislauf 40, zumindest gleich dem Druck nahe dem Y-Stück 22, vgl. Figur 1.
- Der Drucksensor 54 misst den Druck in diesem Segment zwischen den Elementen 34 und 54.
- Ein nicht gezeigter Drucksensor, beispielsweise einer im Beatmungsgerät 1, misst den Druck im Beatmungskreislauf 40.
- Falls die beiden gemessenen Drücke um mehr als eine Toleranz voneinander abweichen, so ist ein Leck L detektiert, und ein entsprechender Alarm wird erzeugt. Ansonsten kann ein Leck ausgeschlossen werden.

**Bezugszeichenliste**

| 1 | Beatmungsgerät, bevorzugt Anästhesiegerät |
|---|---|
| 2 | Messkammer des Sensors 53, im Luftspalt 3 gebildet |
| 3 | Luftspalt, stellt die Messkammer 2 bereit |
| 4, 5 | Feldspulen, erzeugen zusammen mit den Polschuhen 6, 7 ein Magnetfeld in der Messkammer 2 |
| 6, 7 | Polschuhe, erzeugen zusammen mit den Feldspulen 4, 5 ein Magnetfeld in der Messkammer 2 |
| 8 | Thermoelement, umfasst die Drähte 17, 18 und die Stützdrähte 15, 16 |
| 9 | Wandung der Messkammer 2 |
| 10 | Einlass in die Messkammer 2 |
| 11 | Auslass aus der Messkammer 2 |
| 12 | Verbindungsstelle zwischen dem Stützdraht 15 und den Draht 17 |
| 13 | Verbindungsstelle zwischen den Drähten 17 und 18 |
| 14 | Verbindungsstelle zwischen dem Stützdraht 16 und den Draht 18 |
| 15, 16 | Stützdrähte für das Thermoelement 8 |
| 17, 18 | Drähte des Thermoelements 8, sind in der Verbindungsstelle 13 miteinander verbunden |
| 19 | Messwiderstand des Thermoelements 8 |
| 20 | Spannungsquelle |
| 21 | patientenseitige Koppeleinheit in Form eines Mundstücks oder einer Atemmaske, mit dem Y-Stück 22 verbunden |
| 22 | Y-Stück, welche die patientenseitige Koppeleinheit 21 mit einer Zufuhrleitung für die Zufuhr von Gas (Einatmung, Inspiration) und eine Abfuhrleitung für die Abfuhr von Gas (Ausatmung, Exspiration) verbindet |
| 23a | Rückschlagventil, das in der Einatmungs-Leitung 32 einen Gasfluss in Richtung des Patienten P durchlässt und in Gegenrichtung gesperrt |
| 23b | Rückschlagventil, das in der Ausatmungs-Leitung 33 einen Gasfluss weg vom Patienten P durchlässt und in Richtung zum Patienten P hin sperrt |
| 24a | Gebläse, welches einen Volumenfluss in Richtung des Patienten P erzeugt |
| 24b | PEEP-Ventil, welches einen Druck in der Lunge des Patienten P aufrechterhält |
| 25a | Kohlendioxidabsorber, absorbiert aus dem Beatmungskreislauf 40 Kohlendioxid |
| 26 | Atembeutel, über den der Beatmungskreislauf 40 angetrieben werden kann |
| 27 | Fluidstrom von Frischluft oder sonstigem Frischgas zum Beatmungskreislauf 40 |
| 28 | Fluidstrom von dampfförmigem Narkosemittel zum Beatmungskreislauf 40 |
| 29 | einstellbares Überdruckventil, welches Gas aus dem Beatmungskreislauf 40 abzulassen vermag |
| 30 | Signalverarbeitungseinheit für die Sensor-Anordnung 50, wertet Signale von den Sensoren 53, 54, 57 und 58 aus, vermag ein Leck L zu detektieren und eine Nachricht an das Beatmungs-Steuergerät 35 zu übermitteln |

(fortgesetzt)

| | |
|---|---|
| 31 | Narkosemittelverdampfer, erzeugt den Narkosemittelstrom 28 |
| 32 | Atemgasleitung für die Einatmung, mit dem Y-Stück 22 verbunden, weist das Rückschlagventil 23a auf |
| 33 | Atemgasleitung für die Ausatmung, mit dem Y-Stück 22 verbunden, weist das Rückschlagventil 23b auf |
| 34 | Abzweigpunkt des Entnahmeschlauchs 52 |
| 35 | Beatmungs-Steuergerät, steuert die Pumpe 24a und den Narkosemittelverdampfer 31 an, empfängt Signale über die jeweilige Gaskonzentration und Nachrichten von der Signalverarbeitungseinheit 30, generiert bei Bedarf einen Alarm über ein aufgetretenes Leck L |
| 36 | Drucksensor im Beatmungskreislauf 40, misst bevorzugt den am Patienten P anliegenden Druck $P_{aw}$ |
| 37 | Einmündepunkt, in dem der Abführschlauch 56 in den Beatmungskreislauf 40 mündet, flussaufwärts vom Kohlendioxidabsorber 25a angeordnet |
| 40 | Beatmungskreislauf, durch den der Patient P künstlich beatmet wird, umfasst die patientenseitige Koppeleinheit 21, das Y-Stück 22, die Einatmungs-Leitung 32 und die Ausatmungs-Leitung 33 |
| 42 | Leistungsverstärker zwischen der Spannungsquelle 43 und der Feldspule 5 |
| 43 | Spannungsquelle, gibt eine sinusförmige elektrische Spannung ab |
| 44 | optionale Anzeigeeinrichtung des Sensors 53 |
| 50 | Sensor-Anordnung, welche die Konzentration von $O_2$, $CO_2$, $N_2O$ und optional von Narkosemittel misst, umfasst die Sensoren 51, 53 und 54, die Pumpe 55 sowie den Drucksensor 57 |
| 51 | Wasserfalle flussaufwärts von der Sensor-Anordnung 50, umfasst mindestens eine Membrane, bevorzugt aus PTFE, und einen Tank |
| 52 | Entnahmeschlauch, durch den hindurch eine Gasprobe Gp aus dem Beatmungskreislauf 40 entnommen wird, beginnt in einem Abzweigpunkt 34 zwischen der patientenseitigen Koppeleinheit 21 und dem Y-Stück 22 und führt zu der Wasserfalle 51 |
| 53 | Sensor für die $O_2$-Konzentration in der Gasprobe Gp, misst die Konzentration con(50) |
| 54 | Sensor für die Konzentration von $CO_2$, $H_2O$ und Narkosemittel in der Gasprobe Gp |
| 55 | Pumpe, welche eine Gasprobe Gp in den Entnahmeschlauch 52 hinein saugt |
| 56 | Abführschlauch, durch den hindurch eine Gasprobe Gp wieder in den Beatmungskreislauf 40 eingespeist wird, führt zu einem Einmündepunkt 37 flussaufwärts von dem Kohlendioxidabsorber 25a |
| 57 | Drucksensor der Sensor-Anordnung 50, misst den Druck $P_{cell}$ |
| 58 | Sensor für den Umgebungsdruck $P_{amb}$ |
| 62 | Elektromagnet, erzeugt ein zeitlich veränderliches Magnetfeld im Luftspalt 3, umfasst die Spule 63 |
| 63 | elektrische Spule des Elektromagneten 62 |
| 64 | Messeinheit, als Chip implementiert, umfasst das Wärmeleitungs-Messelement 65, das Heizelement 66 und die Membrane 67 |
| 65 | elektrisch steuerbares Wärmeleitungs-Messelement der Messeinheit 64 |
| 66 | elektrisch steuerbares Heizelement der Messeinheit 64 |
| 67 | Membrane der Messeinheit 64 |
| 68 | Messstelle der Messeinheit 64 |
| 69 | Rahmen der Messeinheit 64 |
| 70 | Verstärker |
| 71 | Spannungsteiler |
| 72 | Gleichspannungsquelle |

(fortgesetzt)

| | |
|---|---|
| 73 | Tiefpass-Filter, liefert an seinem Ausgang das Signal 76 |
| 74 | Hochpass-Filter, liefert an seinem Ausgang das Signal 75 |
| 75 | Signal am Ausgang des Hochpass-Filters 74, korreliert mit dem Anteil von Sauerstoff |
| 76 | Signal am Ausgang des Tiefpass-Filters 73, korreliert mit der Wärmeleitfähigkeit |
| 100 | Messsystem, umfasst die Sensor-Anordnung 50, die Signalverarbeitungseinheit 30, die Pumpe 55, den Entnahmeschlauch 52 und den Abführschlauch 56 |
| 200 | Beatmungs-Anordnung, umfasst das Beatmungsgerät 1, das Messsystem 100, die patientenseitige Koppeleinheit 21 und die Atemgasleitungen 32 und 33 |
| con(40) | Konzentration von Sauerstoff im Beatmungskreislauf 40 und damit in der Gasprobe Gp am Abzweigungspunkt 34, wird erfasst oder in einem leckfreien Zustand oder im Beatmungsgerät 1 gemessen, ist bei einem leckfreien Zustand gleich con(50) |
| con(50) | Konzentration von Sauerstoff in der Gasprobe Gp, die die Sensor-Anordnung 50 erreicht, wird vom Sensor 53 gemessen, ist bei einem leckfreien Zustand gleich con(40) |
| con($CO_2$) | Konzentration von $CO_2$ in der Gasprobe Gp, die die Sensor-Anordnung 50 erreicht, wird vom Sensor 53 gemessen |
| con(40, $CO_2$) | Konzentration von $CO_2$ im Beatmungskreislauf 40 und damit in der Gasprobe Gp am Abzweigungspunkt 34 |
| con(env) | Konzentration von Sauerstoff in der Umgebungsluft, wird vorgegeben |
| con(rel) | zweites Maß für den relativen Leck-Volumenfluss Vol'(rel), das abhängig von der Sauerstoff-Konzentration con(50) der Gasprobe Gp ermittelt wird |
| Gg | Gasgemisch, welches das Anästhesiegerät 1 zur patientenseitigen Koppeleinheit 21 fördert |
| Gp | Gasprobe, wird aus dem Beatmungskreislauf 40 abgezweigt, durch den Entnahmeschlauch 52 hindurch zur Sensor-Anordnung 50 geläutet und durch den Abführschlauch 56 wieder in den Beatmungskreislauf 40 eingespeist |
| L | Leck, das sowohl im Intervall Ta als auch im Intervall Tb zwischen den Beatmungskreislauf 40 und der Sensor-Anordnung 50 auftritt und erfindungsgemäß detektiert wird |
| P | Patient, der künstlich beatmet wird und mit der patientenseitigen Koppeleinheit 21 verbunden ist |
| $P_{amb}$ | Druck in der Umgebung der Beatmungs-Anordnung 200, vom Drucksensor 58 gemessen |
| $P_{aw}$ | Druck in der Atemgasleitung 32, vom Drucksensor 58 gemessen |
| $P_{cell}$ | Druck der Gasprobe Gp am Eingang der Sensor-Anordnung 50, vom Drucksensor 57 gemessen |
| Tol | Toleranzband für den Entscheidungs-Zeitraum T(dec) |
| T(L) | Zeitraum, in dem das Leck L aufgetreten ist |
| T(dec) | Entscheidungs-Zeitraum, in dem die Werte der Maße für den relativen Leck-Volumenfluss liegen, die für die Entscheidung über ein Leck verwendet werden |
| T(ref) | vorhergehender Referenz-Zeitraum |
| U | elektrische Spannung zwischen den Stützdrähten 15 und 16 |
| Vol'(40) | Volumenfluss, der aus dem Beatmungskreislauf 40 abgesaugt wird |
| Vol'(50) | zeitlich konstanter gesamter Volumenfluss zur Sensor-Anordnung 50, gleich Vol'(40) + Vol'(env) |
| Vol'(env) | Volumenfluss, der durch ein Leck L hindurch aus der Umgebung zur Sensor-Anordnung 50 fließt |
| Vol'(rel) | relativer Leck-Volumenfluss, Anteil des Volumenflusses Vol'(env) durch einen Leck L am gesamten Volumenfluss Vol'(50) zur Sensor-Anordnung 50 |
| WLF(40) | Wärmeleitfähigkeit der Gasprobe Gp, ist gleich der Wärmeleitfähigkeit im Beatmungskreislauf 40 am Abzweigpunkt 34 |
| WLF(50) | Wärmeleitfähigkeit der Gasprobe Gp, welche die Sensor-Anordnung 50 erreicht, wird vom Sensor 53 gemessen |

(fortgesetzt)

| WLF(env) | Wärmeleitfähigkeit der Umgebungsluft |
|---|---|
| WLF(rel) | erstes Maß für den relativen Leck-Volumenfluss Vol'(rel), das abhängig von der Wärmeleitfähigkeit WLF(50) der Gasprobe Gp ermittelt wird |

**Patentansprüche**

1. Überwachungs-Verfahren zur Überwachung eines Messsystems (100) für eine künstliche Beatmung eines Patienten (P),

   wobei das Messsystem (100) eine Sensor-Anordnung (50) und eine Sensor-Fluidführungseinheit (52, 56) umfasst,
   wobei das Überwachungs-Verfahren durchgeführt wird, während

   - der Patient (P) mit einer patientenseitigen Koppeleinheit (21) verbunden ist und
   - mithilfe einer Patienten-Fluidführungseinheit (32, 33) eine Fluidverbindung (40) zwischen der patientenseitigen Koppeleinheit (21) und einem medizinischen Gerät (1), insbesondere einem Beatmungsgerät, hergestellt ist, und

   wobei das Überwachungs-Verfahren die automatisch durchgeführten Schritte umfasst, dass

   - eine Gasprobe (Gp) aus der Patienten-Fluidführungseinheit (32, 33) abgezweigt und durch die Sensor-Fluidführungseinheit (52, 56) hindurch zu der Sensor-Anordnung (50) geleitet wird und
   - entschieden wird, ob ein Indiz für ein Leck (L) zwischen der Patienten-Fluidführungseinheit (32, 33) und der Sensor-Anordnung (50) aufgetreten ist,

   wobei das Leck (L) eine Fluidverbindung zwischen der Sensor-Fluidführungseinheit (52, 56) und / oder der Sensor-Anordnung (50) einerseits und einer Umgebung des Messsystems (100) andererseits herstellt,
   **dadurch gekennzeichnet, dass**
   das Überwachungs-Verfahren die weiteren Schritte umfasst, dass

   - in der Sensor-Fluidführungseinheit (52, 56) und / oder der Sensor-Anordnung (50) wenigstens zeitweise ein Unterdruck relativ zu einer Umgebung des Messsystems (100) hergestellt wird,
   - unter Verwendung von Messwerten der Sensor-Anordnung (50) ein Wärmeleitfähigkeits-Verlauf, das ist ein zeitlicher Verlauf der Wärmeleitfähigkeit [WLF(50)] der Gasprobe (Gp), welche die Sensor-Anordnung (50) erreicht, ermittelt wird und
   - die Entscheidung, ob ein Indiz für ein Leck (L) zwischen der Patienten-Fluidführungseinheit (32, 33) und der Sensor-Anordnung (50) aufgetreten ist, in Abhängigkeit von einer zeitlichen Veränderung der ermittelten Wärmeleitfähigkeit [WLF(50)] getroffen wird.

2. Überwachungs-Verfahren nach Anspruch 1,

   **dadurch gekennzeichnet, dass**
   das Überwachungs-Verfahren die zusätzlichen Schritte umfasst, dass abhängig von

   - dem Wärmeleitfähigkeits-Verlauf [WLF(50)] und
   - einer vorgegebenen Wärmeleitfähigkeit der Umgebung des Messsystems (100)

   ein zeitlicher Verlauf eines ersten Maßes [WLF(rel)] für einen relativen Leck-Volumenfluss ermittelt wird, wobei der relative Leck-Volumenfluss das Verhältnis zwischen

   - dem bewirkten Volumenfluss durch ein zu detektierendes Leck (L) hindurch und
   - dem gesamten Volumenfluss zur Sensor-Anordnung (50)

   ist, und

die Entscheidung, ob ein Indiz für ein Leck (L) aufgetreten ist,
in Abhängigkeit von einer zeitlichen Veränderung des ersten Maßes [WLF(rel)] gefällt wird.

3. Überwachungs-Verfahren nach Anspruch 2,
   **dadurch gekennzeichnet, dass**

   entschieden wird, dass ein Indiz für ein Leck (L) aufgetreten ist,
   wenn das erste Maß [WLF(rel)] in einem Entscheidungs-Zeitraum [T(dec)], dessen zeitliche Dauer größer als eine vorgegebene untere Zeitdauer-Schranke ist,
   stärker als eine vorgegebene untere Veränderungs-Schranke (Tol) vom ersten Maß [WLF(rel)] in einem Referenz-Zeitraum [T(ref)], der vor dem Entscheidungs-Zeitraum liegt,
   abweicht.

4. Überwachungs-Verfahren nach einem der vorhergehenden Ansprüche,
   **dadurch gekennzeichnet, dass**

   das Überwachungs-Verfahren die zusätzlichen Schritte umfasst, dass zusätzlich unter Verwendung von Messwerten der Sensor-Anordnung (50)

   - ein Konzentrations-Verlauf, das ist ein zeitlicher Verlauf der Konzentration [con(50)] eines Bestandteils ($O_2$) der Gasprobe (Gp), und / oder
   - ein Druck-Verlauf, das ist ein zeitlicher Verlauf des Drucks ($P_{cell}$) der Gasprobe (Gp),

   ermittelt wird und
   die Entscheidung, ob ein Indiz für ein Leck (L) aufgetreten ist,
   zusätzlich in Abhängigkeit von einer zeitlichen Veränderung der ermittelten Konzentration [con(50)] und / oder des Drucks ($P_{cell}$) gefällt wird.

5. Überwachungs-Verfahren nach Anspruch 4,
   **dadurch gekennzeichnet, dass**

   der Bestandteil ($O_2$) der Gasprobe (Gp), dessen Konzentrations-Verlauf gemessen wird, ein paramagnetisches Gas ist und
   das Überwachungs-Verfahren die weiteren Schritte umfasst, dass

   - wenigstens ein Teil der Gasprobe (Gp) in eine Messkammer (2) der Sensor-Anordnung (50) geleitet wird,
   - die Sensor-Anordnung (50) an die Messkammer (2) ein Magnetfeld mit einer oszillierenden Feldstärke anlegt,
   - ein Heizelement (8, 66) erhitzt wird und das erhitzte Heizelement (8, 66) der Gasprobe (Gp) in der Messkammer (2) Wärmeenergie zuführt,
   - eine elektrische Detektionsgröße (U) des Heizelements (8, 66),

     insbesondere die am Heizelement (8, 66) anliegende elektrische Spannung (U) oder die vom Heizelement (8, 66) aufgenommene elektrische Leistung,
     gemessen wird,

   - mittels einer Filterung der elektrischen Detektionsgröße (U)

     ein oszillierendes Signal (75), das abhängig von der Magnetfeldstärke oszilliert, und
     ein weiteres Signal (76), dessen zeitlicher Verlauf nicht von der Oszillation der Magnetfeldstärke abhängt,
     hergeleitet werden,

   - das oszillierende Signal (75) als ein Maß für den Konzentration-Verlauf des paramagnetischen Bestandteils ($O_2$) der Gasprobe (Gp) verwendet wird und
   - das weitere Signal (76) als ein Maß für den Wärmeleitfähigkeits-Verlauf der Gasprobe (Gp) verwendet wird.

6. Überwachungs-Verfahren nach Anspruch 2 und nach Anspruch 4 oder Anspruch 5,

**dadurch gekennzeichnet, dass**

das Überwachungs-Verfahren die weiteren Schritte umfasst, dass abhängig von

- dem Konzentrations-Verlauf [con(50)] des Gasproben-Bestandteils ($O_2$) oder dem Verlauf des Gasproben-Drucks ($P_{cell}$) und
- einer vorgegebenen Soll-Konzentration des Bestandteils ($O_2$) oder eines Drucks ($P_{amb}$) in der Umgebung des Messsystems (100)

ein zeitlicher Verlauf eines zweiten Maß [con(rel)] für den relativen Leck-Volumenfluss berechnet wird, und die Entscheidung, ob ein Indiz für ein Leck (L) aufgetreten ist,

- in Abhängigkeit von der zeitlichen Veränderung des ersten Maßes [WLF(rel)] und
- in Abhängigkeit einer zeitlichen Veränderung des zweiten Maßes [con(rel)] gefällt wird.

7. Überwachungs-Verfahren nach Anspruch 6,
**dadurch gekennzeichnet, dass**
entschieden wird, dass ein Indiz für ein Leck(L) aufgetreten ist, wenn

- das erste Maß [WLF(rel)] in einem Entscheidungs-Zeitraum [T(dec)], dessen zeitliche Dauer größer als eine vorgegebene untere Zeitdauer-Schranke ist,
stärker als eine vorgegebene Veränderungs-Schranke vom erste Maß [WLF(rel)] in einem Referenz-Zeitraum [T(ref)], der vor dem Entscheidungs-Zeitraum [T(dec)] liegt, abweicht,
- auch das zweite Maß [con(rel)] im Entscheidungs-Zeitraum [T(dec)] stärker als die vorgegebene Veränderungs-Schranke vom zweite Maß [con(rel)] im Referenz-Zeitraum [T(ref)] abweicht und
- im Entscheidungs-Zeitraum [T(dec)] die beiden Maße [WLF(rel), con(rel)] um nicht mehr als ein vorgegebenes Toleranzband (Tol) voneinander abweichen.

8. Überwachungs-Verfahren nach einem der vorhergehenden Ansprüche,
**dadurch gekennzeichnet, dass**

dann, wenn entschieden wird, dass ein Indiz für ein Leck (L) aufgetreten ist,
eine Überprüfungs-Abfolge durchgeführt wird, welche die automatisch durchgeführten Schritte umfasst, dass

- der Schritt, die Gasprobe (Gp) durch die Sensor-Fluidführungseinheit (52, 56) zur Sensor-Anordnung (50) zu leiten, unterbrochen wird,
- ein Maß für den Druck ($P_{cell}$) in der Sensor-Anordnung (50) gemessen wird,
- ein Maß für den Druck ($P_{aw}$) in der Patienten-Fluidführungseinheit (32, 33) 33) gemessen wird und
- entschieden wird, dass ein Leck (L) vorliegt, wenn die Abweichung zwischen den beiden gemessenen Drücken ($P_{cell}$, $P_{aw}$) ein vorgegebenes Kriterium erfüllt.

9. Messsystem (100) zur Überwachung einer künstlichen Beatmung eines Patienten (P),

wobei der Patient (P) mit einer patientenseitigen Koppeleinheit (21) verbunden oder wenigstens zeitweise verbindbar ist,
wobei das Messsystem (100) eine Sensor-Anordnung (50) und eine Sensor-Fluidführungseinheit (52, 56) umfasst,
wobei die Sensor-Anordnung (50) eine Signalverarbeitungseinheit (30) umfasst,
wobei mithilfe einer Patienten-Fluidführungseinheit (32, 33) eine Fluidverbindung zwischen der patientenseitige Koppeleinheit (21) und einem medizinischen Gerät (1), insbesondere einem Beatmungsgerät, herstellbar oder wenigstens zeitweise hergestellt ist,
wobei das Messsystem (100) dazu ausgestaltet ist, eine Gasprobe (Gp) aus der Patienten-Fluidführungseinheit (32, 33) abzuzweigen und durch die Sensor-Fluidführungseinheit (52, 56) zur Sensor-Anordnung (50) zu leiten, und
wobei die Signalverarbeitungseinheit (30) dazu ausgestaltet ist,
automatisch zu entscheiden, ob ein Indiz für ein Leck (L) zwischen der Patienten-Fluidführungseinheit (32, 33) und der Sensor-Anordnung (50) aufgetreten ist,
wobei das Leck (L) eine Fluidverbindung zwischen der Sensor-Fluidführungseinheit (52, 56) und / oder der Sensor-Anordnung (50) einerseits und einer Umgebung des Messsystems (100) andererseits herstellt,

**dadurch gekennzeichnet, dass**

die Sensor-Anordnung (50) einen Wärmeleitfähigkeits-Sensor (53) umfasst,

wobei der Wärmeleitfähigkeits-Sensor (53) dazu ausgestaltet ist, einen Wärmeleitfähigkeits-Verlauf, das ist ein zeitlicher Verlauf der Wärmeleitfähigkeit [WLF(50)] der Gasprobe (Gp), welche die Sensor-Anordnung (50) erreicht, zu ermitteln,

wobei das Messsystem (100) dazu ausgestaltet ist, wenigstens zeitweise in der Sensor-Fluidführungseinheit (52, 56) und / oder der Sensor-Anordnung (50) einen Unterdruck relativ zu einer Umgebung des Messsystems (100) herzustellen, und

wobei die Signalverarbeitungseinheit (30) dazu ausgestaltet ist,

die Entscheidung, ob ein Indiz für ein Leck (L) zwischen der Patienten-Fluidführungseinheit (32, 33) und der Sensor-Anordnung (50) aufgetreten ist,

in Abhängigkeit von einer zeitlichen Veränderung des ermittelten Wärmeleitfähigkeits-Verlaufs [WLF(50)] zu treffen.

10. Messsystem (100) nach Anspruch 9,

   **dadurch gekennzeichnet, dass**

   die Signalverarbeitungseinheit (30) zusätzlich dazu ausgestaltet ist, unter Verwendung von Messwerten der Sensor-Anordnung (50)

   - einen Konzentrations-Verlauf, das ist ein zeitlicher Verlauf der Konzentration [con(50)] eines Bestandteils ($O_2$) der Gasprobe (Gp), und / oder
   - einen Druck-Verlauf, das ist ein zeitlicher Verlauf des Drucks ($P_{cell}$) der Gasprobe (Gp),

   zu ermitteln und

   die Entscheidung, ob ein Indiz für ein Leck (L) aufgetreten ist,

   zusätzlich in Abhängigkeit von einer zeitlichen Veränderung der ermittelten Konzentration [con(50)] und / oder des Drucks ($P_{cell}$) zu fällen.

11. Messsystem (100) nach Anspruch 10,
   **dadurch gekennzeichnet, dass**

   der Bestandteil ($O_2$) der Gasprobe (Gp), dessen Konzentrations-Verlauf gemessen wird, ein paramagnetisches Gas ist und

   der Wärmeleitfähigkeits-Sensor (53)

   - eine Messkammer (2),
   - einen Magnetfeld-Erzeuger (4, 5, 6, 7, 62) und
   - ein Heizelement (8, 66)

   umfasst,

   wobei der Magnetfeld-Erzeuger (4, 5, 6, 7, 62) dazu ausgestaltet ist, an die Messkammer (2) ein Magnetfeld mit einer oszillierenden Feldstärke anzulegen,

   wobei die Sensor-Anordnung (50) dazu ausgestaltet ist,

   - die Gasprobe (Gp) oder wenigstens einen Teil der Gasprobe (Gp) in die Messkammer (2) zu leiten und
   - das Heizelement (8, 66) zu erhitzen,

   wobei das erhitzte Heizelement (8, 66) dazu ausgestaltet ist, der Gasprobe (Gp) in der Messkammer (2) Wärmeenergie zuzuführen

   wobei der Wärmeleitfähigkeits-Sensor (53) dazu ausgestaltet ist, eine elektrische Detektionsgröße (U) des Heizelements (8, 66) zu messen,

   insbesondere die am Heizelement (8, 66) anliegende elektrische Spannung (U) oder die vom Heizelement (8, 66) aufgenommene elektrische Leistung,

   wobei die Sensor-Anordnung (50) dazu ausgestaltet ist,

   - mittels einer Filterung der elektrischen Detektionsgröße (U)

ein oszillierendes Signal (75), das abhängig von der Magnetfeldstärke oszilliert, und

ein weiteres Signal (76), dessen zeitlicher Verlauf nicht von der oszillierenden Magnetfeldstärke abhängt,

herzuleiten,

- das oszillierende Signal (75) als ein Maß für den Konzentrations-Verlauf des paramagnetischen Bestandteils ($O_2$) der Gasprobe (Gp) zu verwenden und
- das weitere Signal (76) als ein Maß für den Wärmeleitfähigkeits-Verlauf der Gasprobe (Gp) zu verwenden.

**12.** Beatmungs-Anordnung (200) zur künstlichen Beatmung eines Patienten (P),

wobei die Beatmungs-Anordnung (200)

- ein medizinisches Gerät (1), insbesondere ein Beatmungsgerät,
- eine Patienten-Fluidführungseinheit (32, 33),
- eine patientenseitige Koppeleinheit (21) und
- ein Messsystem (100) nach einem der Ansprüche 9 bis 11

umfasst,

wobei die patientenseitige Koppeleinheit (21) mit dem Patienten (P) verbunden oder wenigstens zeitweise verbindbar ist,

wobei die Beatmungs-Anordnung (200) dazu ausgestaltet, ein Gasgemisch (Gg) vom medizinischen Gerät (1) durch die Patienten-Fluidführungseinheit (32, 33) hindurch zur patientenseitigen Koppeleinheit (21) zu fördern, und

wobei das Messsystem (100) dazu ausgestaltet, ein Indiz für ein Leck (L) zwischen der Patienten-Fluidführungseinheit (32, 33) und der Sensor-Anordnung (50) zu detektieren.

## Claims

**1.** Monitoring method for monitoring a measuring system (100) for artificial ventilation of a patient (P),

the measuring system (100) comprising a sensor arrangement (50) and a sensor fluid guide unit (52, 56), the method being carried out while

- the patient (P) is connected to a patient-side coupling unit (21) and
- a fluid connection (40) is established between the patient-side coupling unit (21) and a medical device (1), in particular a ventilator, by means of a patient fluid guide unit (32, 33), and

the method comprising the automatically carried out steps whereby

- a gas sample (Gp) is branched off from the patient fluid guide unit (32, 33) and passed through the sensor fluid guide unit (52, 56) to the sensor arrangement (50) and
- it is decided whether an indication of a leak (L) has occurred between the patient fluid guide unit (32, 33) and the sensor arrangement (50),

the leak (L) establishing a fluid connection between the sensor fluid guide unit (52, 56) and/or the sensor arrangement (50) on the one hand and an environment of the measuring system (100) on the other hand,

**characterized in that**

the monitoring method comprises the further steps whereby

- a negative pressure is created at least temporarily relative to an environment of the measuring system (100) in the sensor fluid guide unit (52, 56) and/or the sensor arrangement (50),
- a thermal conductivity curve, i.e. a temporal curve of the thermal conductivity [WLF(50)] of the gas sample (Gp) which reaches the sensor arrangement (50) is determined using measured values of the sensor arrangement (50) and
- Itit is decided whether an indication of a leak (L) has occurred between the patient fluid guide unit (32, 33) and the sensor arrangement (50) depending on a temporal change in the determined thermal conductivity [WLF(50)] over time.

2. Monitoring method according to claim 1,
**characterized in that**

the method comprises the additional steps whereby
depending on

- the thermal conductivity curve [WLF(50)] and
- a specified thermal conductivity of the environment of the measuring system (100)

a time course of a first measure [WLF(rel)] for a relative leak volume flow is determined,
the relative leak volume flow being the ratio between

- the volume flow caused by a leak (L) to be detected and
- the total volume flow to the sensor arrangement (50), and

the decision as to whether an indication of a leak (L) has occurred
is made depending on a temporal change of the first measure [WLF(rel)].

3. Monitoring method according to claim 2,
**characterized in that**

it is decided that an indication of a leak (L) has occurred,
if the first measure [WLF(rel)] in a decision period [T(dec)], the duration of which is greater than a specified lower duration bound,
deviates more than a specified lower change bound (Tol)
from the first measure [WLF(rel)] in a reference period [T(ref)] that lies before the decision period

4. Monitoring method according to any of the preceding claims,
**characterized in that**

the method comprises the additional steps whereby
additionally using measured values of the sensor arrangement (50)

- a concentration curve, i.e. a temporal curve of the concentration [con(50)] of a component ($O_2$) of the gas sample (Gp), and / or
- a pressure curve, i.e. a temporal curve of the pressure ($P_{cell}$) of the gas sample (Gp),

is determined and
the decision as to whether an indication of a leak (L) has occurred
is made additionally depending on a temporal change of the determined concentration [con(50)] and / or the pressure ($P_{cell}$).

5. Monitoring method according to claim 4,
**characterized in that**

the component ($O_2$) of the gas sample (Gp), the concentration curve of which is measured, is a paramagnetic gas and
the monitoring method comprises the further steps whereby

- at least a part of the gas sample (Gp) is conducted into a measuring chamber (2) of the sensor arrangement (50),
- the sensor arrangement (50) applies a magnetic field having an oscillating field strength to the measuring chamber (2),
- a heating element (8, 66) is heated and the heated heating element (8, 66) supplies heat energy to the gas sample (Gp) in the measuring chamber (2),

- an electrical detection variable (U) of the heating element (8, 66),

in particular the electrical voltage (U) applied to the heating element (8, 66) or the electrical power absorbed by the heating element (8, 66),
is measured,

- by means of filtering the electrical detection variable (U)

an oscillating signal (75) which oscillates depending on the magnetic field strength, and
a further signal (76), the temporal curve of which does not depend on the oscillation of the magnetic field strength, are derived,

- the oscillating signal (75) is used as a measure for the concentration profile of the paramagnetic component $(O_2)$ of the gas sample (Gp) and
- the further signal (76) is used as a measure for the thermal conductivity curve of the gas sample (Gp).

6. Monitoring method according to claim 2 and claim 4 or claim 5,
**characterized in that**

the monitoring method comprises the further steps whereby
depending on

- the concentration curve [con(50)] of the gas sample component $(O_2)$ or the curve of the gas sample pressure $(P_{cell})$ and
- a specified target concentration of the component $(O_2)$ or a pressure $(P_{amb})$ in the environment of the measuring system (100)

a temporal curve of a second measure [con(rel)] for the relative leak volume flow is calculated, and
the decision as to whether an indication of a leak (L) has occurred is made

- depending on the temporal change of the first measure [WLF(rel)] and
- depending on a temporal change of the second measure [con(rel)].

7. Monitoring method according to claim 6,
**characterized in that**

it is decided that an indication of a leak (L) has occurred if

- the first measure [WLF(rel)] in a decision period [T(dec)], the duration of which is greater than a specified lower duration bound,

deviates more than a specified change bound from the first measure [WLF(rel)] in a reference period [T(ref)] that lies before the decision period [T(dec)],

- the second measure [con(rel)] in the decision period [T(dec)] also deviates more than the specified change bound from the second measure [con(rel)] in the reference period [T(ref)] and
- in the decision period [T(dec)] the two dimensions [WLF(rel), con(rel)] do not deviate from each other by more than a specified tolerance band (Tol).

8. Monitoring method according to any of the preceding claims,
**characterized in that**

when it is then decided that an indication of a leak (L) has occurred,
a verification sequence is carried out which comprises the automatically carried out steps whereby

- the step of conducting the gas sample (Gp) through the sensor fluid guide unit (52, 56) to the sensor arrangement (50) is interrupted,
- a measure for the pressure $(P_{cell})$ is measured in the sensor arrangement (50),

- a measure for the pressure ($P_{aw}$) is measured in the patient fluid guide unit (32, 33) 33) and
- it is decided that there is a leak (L) if the deviation between the two measured pressures ($P_{cell}$, $P_{aw}$) meets a specified criterion.

9. Measuring system (100) for monitoring artificial ventilation of a patient (P),

the patient (P) being connected or at least temporarily connectable to a patient-side coupling unit (21),
the measuring system (100) comprising a sensor arrangement (50) and a sensor fluid guide unit (52, 56),
the sensor arrangement (50) comprising a signal processing unit (30),
a fluid connection between the patient-side coupling unit (21) and a medical device (1), in particular a ventilator, being able to be established or being at least temporarily established by means of a patient fluid guide unit (32, 33),
the measuring system (100) being designed to divert a gas sample (Gp) from the patient fluid guide unit (32, 33) and to guide it through the sensor fluid guide unit (52, 56) to the sensor arrangement (50), and
the signal processing unit (30) being designed to
automatically decide whether an indication of a leak (L) has occurred between the patient fluid guide unit (32, 33) and the sensor arrangement (50),
the leak (L) establishing a fluid connection between the sensor fluid guide unit (52, 56) and/or the sensor arrangement (50) on the one hand and an environment of the measuring system (100) on the other hand,
**characterized in that**
the sensor arrangement (50) comprises a thermal conductivity sensor (53),
the thermal conductivity sensor (53) being designed to determine a thermal conductivity curve, i.e. a temporal curve of the thermal conductivity [WLF(50)] of the gas sample (Gp) which reaches the sensor arrangement (50),
the measuring system (100) being designed to at least temporarily establish a negative pressure in the sensor fluid guide unit (52, 56) and/or the sensor arrangement (50) relative to an environment of the measuring system (100), and
the signal processing unit (30) being designed to
make the decision as to whether an indication of a leak (L) has occurred between the patient fluid guide unit (32, 33) and the sensor arrangement (50),
depending on a temporal change in the determined thermal conductivity curve [WLF(50)].

10. Measuring system (100) according to claim 9,
**characterized in that**

the signal processing unit (30) is additionally designed to use measured values of the sensor arrangement (50) to determine

- a concentration curve, i.e. a temporal curve of the concentration [con(50)] of a component ($O_2$) of the gas sample (Gp), and / or
- a pressure curve, which is a temporal curve of the pressure ($P_{cell}$) of the gas sample (Gp),

and
to make the decision as to whether an indication of a leak (L) has occurred
additionally depending on a temporal change of the determined concentration [con(50)] and / or the pressure ($P_{cell}$).

11. Measuring system (100) according to claim 10,
**characterized in that**

the component ($O_2$) of the gas sample (Gp), the concentration curve of which is measured, is a paramagnetic gas and
the thermal conductivity sensor (53) comprises

- a measuring chamber (2),
- a magnetic field generator (4, 5, 6, 7, 62) and
- a heating element (8, 66),

the magnetic field generator (4, 5, 6, 7, 62) being designed to apply a magnetic field having an oscillating field strength to the measuring chamber (2),
the sensor arrangement (50) being designed to

- direct the gas sample (Gp) or at least a part of the gas sample (Gp) into the measuring chamber (2) and
- to heat the heating element (8, 66),

the heated heating element (8, 66) being designed to supply heat energy to the gas sample (Gp) in the measuring chamber (2)
the thermal conductivity sensor (53) being designed to measure an electrical detection variable (U) of the heating element (8, 66),
in particular the electrical voltage (U) applied to the heating element (8, 66) or the electrical power absorbed by the heating element (8, 66),
the sensor arrangement (50) being designed to

- by means of filtering the electrical detection variable (U)

derive an oscillating signal (75) which oscillates depending on the magnetic field strength, and
a further signal (76), the temporal curve of which does not depend on the oscillating magnetic field strength,

- use the oscillating signal (75) as a measure for the concentration profile of the paramagnetic component ($O_2$) of the gas sample (Gp) and
- use the further signal (76) as a measure for the thermal conductivity curve of the gas sample (Gp).

12. Ventilation arrangement (200) for artificial ventilation of a patient (P),

wherein the ventilation arrangement (200) comprises

- a medical device (1), in particular a ventilator,
- a patient fluid guide unit (32, 33),
- a patient-side coupling unit (21) and
- a measuring system (100) according to any of claims 9 to 11,

wherein the patient-side coupling unit (21) is connected or at least temporarily connectable to the patient (P),
wherein the ventilation arrangement (200) is designed to convey a gas mixture (Gg) from the medical device (1) through the patient fluid guide unit (32, 33) to the patient-side coupling unit (21), and
wherein the measuring system (100) is designed to detect an indication of a leak (L) between the patient fluid guide unit (32, 33) and the sensor arrangement (50).

**Revendications**

1. Procédé de surveillance pour la surveillance d'un système de mesure (100) pour une respiration artificielle d'un patient (P),

dans lequel le système de mesure (100) comprend un agencement de capteurs (50) et une unité de guidage de fluide pour capteur (52, 56),
dans lequel le procédé de surveillance est exécuté pendant que

- le patient (P) est relié à une unité de couplage côté patient (21) et
- que, à l'aide d'une unité de guidage de fluide pour patient (32, 33), une liaison fluidique (40) est établie entre l'unité de couplage côté patient (21) et un appareil médical (1), en particulier un appareil de ventilation, et

dans lequel le procédé de surveillance comprend les étapes exécutées automatiquement, selon lesquelles

- un échantillon de gaz (Gp) est dérivé depuis l'unité de guidage de fluide pour patient (32, 33) et conduit à travers l'unité de guidage de fluide pour capteur (52, 56) jusqu'à l'agencement de capteurs (50) ; et
- on décide si une indication d'une fuite (L) est apparue entre l'unité de guidage de fluide pour patient (32, 33) et l'agencement de capteurs (50),

dans lequel la fuite (L) établit une liaison fluidique entre l'unité de guidage de fluide pour capteur (52, 56) et/ou l'agencement de capteurs (50) d'une part et un environnement du système de mesure (100) d'autre part, **caractérisé en ce que**
le procédé de surveillance comprend les étapes supplémentaires selon lesquelles

- dans l'unité de guidage de fluide pour capteur (52, 56) et/ou l'agencement de capteurs (50), une dépression est créée au moins temporairement par rapport à un environnement du système de mesure (100),
- en utilisant des valeurs de mesure de l'agencement de capteurs (50), une évolution de conductivité thermique est déterminée, laquelle est une évolution temporelle de la conductivité thermique [WLF(50)] de l'échantillon de gaz (Gp) qui atteint l'agencement de capteurs (50), et
- la décision de savoir si une indication d'une fuite (L) est apparue entre l'unité de guidage de fluide pour patient (32, 33) et l'agencement de capteurs (50) est prise en fonction d'une variation temporelle de la conductivité thermique [WLF(50)] déterminée.

2. Procédé de surveillance selon la revendication 1,
**caractérisé en ce que**

le procédé de surveillance comprend les étapes supplémentaires selon lesquelles
en fonction

- de l'évolution de conductivité thermique [WLF(50)] et
- d'une conductivité thermique prédéfinie de l'environnement du système de mesure (100)

une évolution temporelle d'une première mesure [WLF(rel)] pour un débit volumique de fuite relatif est déterminée,
dans lequel le débit volumique de fuite relatif est le rapport entre

- le débit volumique provoqué à travers une fuite (L) à détecter et
- le débit volumique total vers l'agencement de capteurs (50),

et
la décision de savoir si une indication d'une fuite (L) est apparue
est prise en fonction d'une variation temporelle de la première mesure [WLF(rel)].

3. Procédé de surveillance selon la revendication 2,
**caractérisé en ce que**

il est décidé qu'une indication d'une fuite (L) est apparue
lorsque la première mesure [WLF(rel)], dans une période de décision [T(dec)] dont la durée temporelle est supérieure à une limite inférieure de durée prédéfinie, s'écarte plus de la première mesure [WLF(rel)] dans une période de référence [T(ref)] antérieure à la période de décision qu'une limite inférieure de variation (Tol) prédéfinie.

4. Procédé de surveillance selon l'une des revendications précédentes,
**caractérisé en ce que**

le procédé de surveillance comprend les étapes supplémentaires selon lesquelles
en utilisant en outre des valeurs de mesure de l'agencement de capteurs (50)

- une évolution de concentration qui est une évolution temporelle de la concentration [con(50)] d'un composant ($O_2$) de l'échantillon de gaz (Gp), et/ou
- une évolution de pression qui est une évolution temporelle de la pression (Pcell) de l'échantillon de gaz (Gp)

sont déterminées et
la décision de savoir si une indication d'une fuite (L) est apparue
est en outre prise en fonction d'une variation temporelle de la concentration [con(50)] et/ou de la pression ($P_{cell}$) déterminées.

**5.** Procédé de surveillance selon la revendication 4,
**caractérisé en ce que**

le composant (O$_2$) de l'échantillon de gaz (Gp) duquel l'évolution de concentration est mesurée est un gaz paramagnétique et
le procédé de surveillance comprend les étapes supplémentaires selon lesquelles

- au moins une partie de l'échantillon de gaz (Gp) est dirigée vers une chambre de mesure (2) de l'agencement de capteurs (50),
- l'agencement de capteurs (50) applique à la chambre de mesure (2) un champ magnétique comportant une intensité de champ oscillante,
- un élément chauffant (8, 66) est chauffé et l'élément chauffant (8, 66) chauffé fournit de l'énergie thermique à l'échantillon de gaz (Gp) dans la chambre de mesure (2),
- une grandeur de détection électrique (U) de l'élément chauffant (8, 66),

en particulier la tension électrique (U) appliquée à l'élément chauffant (8, 66) ou la puissance électrique absorbée par l'élément chauffant (8, 66),
est mesurée,

- au moyen d'un filtrage de la grandeur de détection électrique (U)

un signal oscillant (75) qui oscille en fonction de l'intensité de champ magnétique, et
un autre signal (76) dont l'évolution temporelle ne dépend pas de l'oscillation de l'intensité de champ magnétique,
peuvent être déduits,

- le signal oscillant (75) est utilisé comme une mesure pour l'évolution de concentration du composant (O$_2$) paramagnétique de l'échantillon de gaz (Gp) et
- l'autre signal (76) est utilisé comme une mesure pour l'évolution de conductivité thermique de l'échantillon de gaz (Gp).

**6.** Procédé de surveillance selon la revendication 2 et selon la revendication 4 ou la revendication 5,
**caractérisé en ce que**

le procédé de surveillance comprend les étapes supplémentaires selon lesquelles
en fonction

- de l'évolution de concentration [con(50)] du composant d'échantillon de gaz (O$_2$) ou de l'évolution de la pression d'échantillon de gaz (Pcell) et
- d'une concentration de consigne prédéfinie du composant (O$_2$) ou d'une pression (P$_{amb}$) dans l'environnement du système de mesure (100)

une évolution temporelle d'une seconde mesure [con(rel)] pour le débit volumique de fuite relatif est calculée, et
la décision de savoir si une indication d'une fuite (L) est apparue est prise

- en fonction de la variation temporelle de la première mesure [WLF(rel)] et
- en fonction d'une variation temporelle de la seconde mesure [con(rel)].

**7.** Procédé de surveillance selon la revendication 6,
**caractérisé en ce que**
il est décidé qu'une indication d'une fuite (L) est apparue lorsque

- la première mesure [WLF(rel)], dans une période de décision [T(dec)] dont la durée temporelle est supérieure à une limite inférieure de durée prédéfinie, s'écarte plus de la première mesure [WLF(rel)] dans une période de référence [T(ref)] antérieure à la période de décision [T(dec)] qu'une limite de variation prédéfinie,
- la seconde mesure [con(rel)], dans la période de décision [T(dec)], s'écarte également plus de la seconde mesure [con(rel)] dans la période de référence [T(ref)] que la limite de variation prédéfinie, et
- dans la période de décision [T(dec)], les deux mesures [WLF(rel), con(rel)] ne s'écartent pas de plus d'une bande de tolérance (Tol) prédéfinie.

8. Procédé de surveillance selon l'une des revendications précédentes,
**caractérisé en ce que**

lorsqu'il est décidé qu'une indication d'une fuite (L) est apparue,
une séquence de vérification est exécutée, laquelle comprend les étapes exécutées automatiquement selon lesquelles

- l'étape consistant à conduire l'échantillon de gaz (Gp) à travers l'unité de guidage de fluide pour capteur (52, 56) jusqu'à l'agencement de capteurs (50) est interrompue,
- une mesure de la pression (Pcell) dans l'agencement de capteurs (50) est mesurée,
- une mesure de la pression (Paw) dans l'unité de guidage de fluide pour patient (32, 33) 33) est mesurée et
- il est décidé qu'il existe une fuite (L) lorsque l'écart entre les deux pressions mesurées (Pcell, $P_{aw}$) satisfait à un critère prédéfini.

9. Système de mesure (100) pour la surveillance d'une ventilation artificielle d'un patient (P),

dans lequel le patient (P) est relié ou peut être relié au moins temporairement à une unité de couplage côté patient (21),
dans lequel le système de mesure (100) comprend un agencement de capteurs (50) et une unité de guidage de fluide pour capteur (52, 56),
dans lequel l'agencement de capteurs (50) comprend une unité de traitement de signaux (30),
dans lequel, à l'aide d'une unité de guidage de fluide pour patient (32, 33), une liaison fluidique entre l'unité de couplage côté patient (21) et un appareil médical (1), en particulier un appareil de ventilation, peut être établie ou est établie au moins temporairement,
dans lequel le système de mesure (100) est conçu pour dériver un échantillon de gaz (Gp) de l'unité de guidage de fluide pour patient (32, 33) et pour le conduire à travers l'unité de guidage de fluide pour capteur (52, 56) jusqu'à l'agencement de capteurs (50), et
dans lequel l'unité de traitement de signaux (30) est configurée pour
décider automatiquement si une indication d'une fuite (L) est apparue entre l'unité de guidage de fluide pour patient (32, 33) et l'agencement de capteurs (50),
dans lequel la fuite (L) établit une liaison fluidique entre l'unité de guidage de fluide pour capteur (52, 56) et/ou l'agencement de capteurs (50) d'une part et un environnement du système de mesure (100) d'autre part,
**caractérisé en ce que**
l'agencement de capteurs (50) comprend un capteur de conductivité thermique (53),
dans lequel le capteur de conductivité thermique (53) est configuré pour déterminer une évolution de conductivité thermique qui est une évolution temporelle de la conductivité thermique [WLF(50)] de l'échantillon de gaz (Gp) qui atteint l'agencement de capteurs (50),
dans lequel le système de mesure (100) est conçu pour établir, au moins temporairement, dans l'unité de guidage de fluide pour capteur (52, 56) et/ou dans l'agencement de capteurs (50), une dépression par rapport à un environnement du système de mesure (100), et
dans lequel l'unité de traitement de signaux (30) est configurée pour
prendre la décision de savoir si une indication d'une fuite (L) est apparue entre l'unité de guidage de fluide pour patient (32, 33) et l'agencement de capteurs (50) en fonction d'une variation temporelle de l'évolution de conductivité thermique [WLF(50)] déterminée.

10. Système de mesure (100) selon la revendication 9,

**caractérisé en ce que**
l'unité de traitement de signal (30) est en outre configurée pour déterminer, en utilisant des valeurs de mesure de l'agencement de capteurs (50),

- une évolution de concentration qui est une évolution temporelle de la concentration [con(50)] d'un composant (O2) de l'échantillon de gaz (Gp), et/ou
- une évolution de pression qui est une évolution temporelle de la pression (Pcell) de l'échantillon de gaz (Gp),

et
la décision de savoir si une indication d'une fuite (L) est apparue
est prise en outre en fonction d'une variation temporelle de la concentration [con(50)] et/ou de la pression ($P_{cell}$)

déterminées.

11. Système de mesure (100) selon la revendication 10,
**caractérisé en ce que**

le composant ($O_2$) de l'échantillon de gaz (Gp) duquel l'évolution de concentration est mesurée est un gaz paramagnétique et
le capteur de conductivité thermique (53) comprend

- une chambre de mesure (2),
- un générateur de champ magnétique (4, 5, 6, 7, 62) et
- un élément chauffant (8, 66),

dans lequel le générateur de champ magnétique (4, 5, 6, 7, 62) est configuré pour appliquer à la chambre de mesure (2) un champ magnétique comportant une intensité de champ oscillante,
dans lequel l'agencement de capteurs (50) est configuré pour

- conduire l'échantillon de gaz (Gp) ou au moins une partie de l'échantillon de gaz (Gp) jusque dans la chambre de mesure (2) et
- chauffer l'élément chauffant (8, 66),

dans lequel l'élément chauffant (8, 66) chauffé est conçu pour fournir de l'énergie thermique à l'échantillon de gaz (Gp) dans la chambre de mesure (2)
dans lequel le capteur de conductivité thermique (53) est configuré pour mesurer une grandeur de détection électrique (U) de l'élément chauffant (8, 66),
en particulier la tension électrique (U) appliquée à l'élément chauffant (8, 66) ou la puissance électrique absorbée par l'élément chauffant (8, 66),
dans lequel l'agencement de capteurs (50) est configuré pour

- au moyen d'un filtrage de la grandeur de détection électrique (U)

déduire un signal oscillant (75) qui oscille en fonction de l'intensité de champ magnétique, et
déduire un autre signal (76) dont l'évolution temporelle ne dépend pas de l'intensité de champ magnétique oscillante,

- utiliser le signal oscillant (75) comme une mesure pour l'évolution de concentration du composant ($O_2$) paramagnétique de l'échantillon de gaz (Gp) et
- utiliser l'autre signal (76) comme une mesure pour l'évolution de conductivité thermique de l'échantillon de gaz (Gp).

12. Agencement de ventilation (200) pour la respiration artificielle d'un patient (P),

dans lequel l'agencement de ventilation (200) comprend

- un appareil médical (1), en particulier un appareil de ventilation,
- une unité de guidage de fluide pour patient (32, 33),
- une unité de couplage côté patient (21) et
- un système de mesure (100) selon l'une des revendications 9 à 11,

dans lequel l'unité de couplage côté patient (21) peut être reliée ou est reliée au moins temporairement au patient (Pt),
dans lequel l'agencement de ventilation (200) est conçu pour transporter un mélange de gaz (Gg) depuis l'appareil médical (1), à travers l'unité de guidage de fluide pour patient (32, 33) et jusqu'à l'unité de couplage côté patient (21), et
dans lequel le système de mesure (100) est configuré pour détecter une indication d'une fuite (L) entre l'unité de guidage de fluide pour patient (32, 33) et l'agencement de capteurs (50).

FIG. 1

**FIG. 2**

EP 4 349 388 B1

**FIG. 3**

FIG. 4

FIG. 5

FIG. 6

FIG. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102020002570 A1
- DE 102010014883 A1
- DE 102020117607 A1
- DE 102007046533 B3
- DE 102009024040 A1